# EUROPEAN PATENT SPECIFICATION

(11) **EP 1 469 077 B1**
(45) Date of publication and mention of the grant of the patent: **16.02.2011**
(21) Application number: 02790914.2
(22) Date of filing: 26.12.2002
(51) Int. Cl.: C12N 15/64, C12P 21/00, C12Q 1/02, A61K 48/00, A61P 35/00, A61P 43/00

(54) **CELL-SPECIFIC EXPRESSION/REPLICATION VECTOR**
ZELLSPEZIFISCHER EXPRESSIONS-/REPLIKATIONSVEKTOR
VECTEUR D'EXPRESSION/REPLICATION SPECIFIQUE DE CELLULE

(30) Priority: 28.12.2001 JP 2001402102; 30.08.2002 JP 2002255395
(43) Date of publication of application: 20.10.2004
(73) Proprietor: Japan Science and Technology Agency, Kawaguchi-shi, Saitama 332-0012 (JP)
(72) Inventor: TAKAHASHI, Katsuhito, Ikeda-shi, Osaka 563-0055 (JP); YAMAMURA, Hisako, Ikoma-shi, Nara 630-0247 (JP)
(74) Representative: Harding, Charles Thomas
(86) International application number: PCT/JP2002/013683
(87) International publication number: WO 2003/057888

(56) References cited:
- WO-A1-02/92816
- US-A- 5 728 379
- AKYUREK LEVENT M ET AL: "SM22alpha promoter targets gene expression to vascular smooth muscle cells in vitro and in vivo" MOLECULAR MEDICINE (NEW YORK), vol. 6, no. 11, November 2000 (2000-11), pages 983-991, XP008066077 ISSN: 1076-1551
- MIYATAKE SHIN-ICHI ET AL: "Inhibition of rat vascular smooth muscle cell proliferation in vitro and in vivo by recombinant replication-competent herpes simplex virus" STROKE, vol. 30, no. 11, November 1999 (1999-11), pages 2431-2439, XP002388481 ISSN: 0039-2499
- CHEON J. ET AL.: 'Adenovirus-mediated suicide-gene therapoy using the herpes simplex virus thymidine kinase gene in cell and animal models of human protate cancer: changes in tumour cell proliferative activity' BJU INT. vol. 85, no. 6, April 2000, pages 759 - 766, XP002967007
- TAKAHASHI K. ET AL.: 'Transcriptional targeting of replication-competent herpes simplex virus to proliferating smooth muscle cells' CIRCULATION vol. 102, no. 18, October 2000, page II-86, 416, XP002967008
- TAKAHASHI K. ET AL.: 'The 5'-flanking region of the human smooth muscle cell calponin gene contains a cis-acting domain for interaction with a methylated DNA-binding transcription repressor' J. BIOCHEM. vol. 120, no. 1, July 1996, TOKYO, pages 18 - 21, XP002967009
- YAMAMURA H. ET AL.: 'Identification of the transcriptional regulatory sequences of human calponin promoter and their use in targeting a conditionally replicating herpes vector to malignant human soft tissue and bone tumors' CANCER RES. vol. 61, no. 10, 15 May 2001, pages 3969 - 3977, XP002967010
- KRUGER M. ET AL.: 'Involvement of proteasome alpha-subunit PSMA7 in hepatitis C virus internal ribosome entry site-mediated translation' MOL. CELL. BIOL. vol. 21, no. 24, December 2001, pages 8357 - 8364, XP002967011
- MARTUZA R.L.: "Conditionally replicating herpes vectors for cancer therapy." J. CLINICAL INVESTIGATION, vol. 105, no. 7, April 2000 (2000-04), pages 841-846, XP002453237
- KURODA T. ET AL: "Development of oncolytic herpes simplex virus (HSV) vector selectively replicating in colorectal cancer cells with highly activated beta-catenin/Wnt pathway." ONCOLYTIC VIRUSES AS CANCER THERAPEUTICS, 9 March 2005 (2005-03-09), - 13 March 2005 (2005-03-13)
- MINETA T. ET AL: "Attenuated multi-mutated herpes simplex virus-1 for the treatment of malignant gliomas." NATURE MEDICINE, vol. 1, no. 9, September 1995 (1995-09), pages 938-943, XP002067917
- HUNTER W.D. ET AL: "Attenuated, replication-competent herpes simplex virus type 1 mutant G207: safety evaluation of intracerebral injection in nonhuman primates." J. VIROL., vol. 73, no. 8, August 1999 (1999-08), pages 6319-6326,

### SEQUENCE LISTING

<110> JAPAN SCIENCE AND TECHNOLOGY CORPORATION
<120> Cell specific express replication vector
<130> B08-01PCT
<140>
   <141>
<150> JP P2001-402102
   <151> 2001-12-28
<150> JP P2002-255395
   <151> 2002-08-30
<160> 9
<170> PatentIn Ver. 2.1
<210> 1
   <211> 41
   <212> DNA
   <213> Homo sapiens
<400> 1
   gaaacaatga cacaatcagc tcccaatacc aagggcctga c 41
<210> 2
   <211> 260
   <212> DNA
   <213> Homo sapiens
<400> 2
   gaaacaatga cacaatcagc tcccaatacc aagggcctga catcacaagg ggaggggaag 60
   gcagctgagg ttgtgggggg aggtgccccg ccccttggca ggcccctaca gccaatggaa 120
   cggccctgga agagacccgg gtcgcctccg gagcttcaaa aacatgtgag gagggaagag 180
   tgtgcagacg gaacttcagc cgctgcctct gttctcagcg tcagtgccgc cactgccccc 240
   gccagagccc accggccagc 260
<210> 3
   <211> 333
   <212> DNA
   <213> Artificial Sequence
<220>
   <223> Description of Artificial Sequence: Region consist of human calponin gene promoter and its structural gene fragment
<400> 3
   gaaacaatga cacaatcagc tcccaatacc aagggcctga catcacaagg ggaggggaag 60
   gcagctgagg ttgtgggggg aggtgccccg ccccttggca ggcccctaca gccaatggaa 120
   cggccctgga agagacccgg gtcgcctccg gagcttcaaa aacatgtgag gagggaagag 180
   tgtgcagacg gaacttcagc cgctgcctct gttctcagcg tcagtgccgc cactgccccc 240
   gccagagccc accggccagc atgtcctctg ctcacttcaa ccgaggccct gcctacgggc 300
   tgtcagccga ggttaagaac aaggtagggg tgg 333
<210> 4
   <211> 445
   <212> DNA
   <213> Homo sapiens
<400>
   gtgagtgcag cgcgcccccg tcccgggtac ctccggttga atctggtggc ttgcaccgac 60
   cccctcccct gtccccagac ggatctagat ggttcttccc tccatcccgt accgacgact 120
   gtcccccctt cccccacccc ctccccggca cattgtcctt ccctcctttc tttgaagaaa 180
   gccgacccgc ccctcactcc gtcacgaggg tgggtgactc agcgtcctcc ttccccgcgg 240
   cgccagaagc cagttgcaac cggtttctga agtaatgtgc aggactcctt acatcagctc 300
   ctctgagtct cgtgattcag ccttgcctcc ctctctcccc ctttgccccc tccccgtccc 360
   acccttaggc gctgggagaa gggagggtgg ggaggtcagg ggcctctcag aggggcctca 420
   cttgttaacc cagcccccat ttcag 445
<210> 5
   <211> 455
   <212> DNA
   <213> Homo sapiens
<400> 5
   ggatcccatg tcccatcaga gctaaaagcc ccaggaggag agggtggctg gtttgtcccc 60
   acaaacccct gggattcccg gctccccagc cccttgcccc tctctccagc cagactctat 120
   tgaactcccc ctcttctcaa actcggggcc agagaacagt gaagtaggag cagccgtaag 180
   tccgggcagg gtcctgtcca taaaaggctt ttcccgggcc ggctccccgc cggcagcgtg 240
   ccccgccccg gcccgctcca tctccaaagc atgcagagaa tgtctcggca gccccggtag 300
   actgctccaa cttggtgtct ttccccaaat atggagcctg tgtggagtca ctgggggagc 360
   cgggggtggg gagcggagcc ggcttcctct agcagggagg gggccgagga gcgagccagt 420
   gggggaggct gacatcacca cggcggcagc ccttt 455
<210> 6
   <211> 24
   <212> DNA
   <213> Artificial Sequence
<220>
   <223> Description of Artificial Sequence:FP1
<400> 6
   gagtgtgcag acggaacttc agcc 24
<210> 7
   <211> 21
   <212> DNA
   <213> Artificial Sequence
<220>
   <223> Description of Artificial Sequence:RP1
<400> 7
   gtctgtgccc aacttggggt c 21
<210> 8
   <211> 20
   <212> DNA
   <213> Artificial Sequence
<220>
   <223> Description of Artificial Sequence:FP2
<400> 8
   cccatcacca tcttccagga 20
<210> 9
   <211> 21
   <212> DNA
   <213> Artificial Sequence
<220>
   <223> Description of Artificial Sequence:RP2
<400> 9
   ttgtcatacc aggaaatgag c 21

## Description

### Technical Field

The present description relates to a cell-specific expression/replication vector that is capable of specifically expressing a gene in a specific cell and does not act on adult normal cells that self-replicate, particularly, to a cell-specific expression/replication vector that is capable of suppressing expression/replication at a desired period after its expression/replication, further to a method for expressing a gene in a specific cell in a living organism by using the vector, or a method for disrupting a specific cell by using the vector, etc. For more details regarding the description, it relates to: (1) construction of a cell-specific expression/replication vector with high safety in the field of gene therapy for cancer, wherein an expression/replication vector that can express genes cell-specifically to specifically disrupt a particular cancer cell itself or a proliferating smooth muscle cell in the new tumor blood vessel is generated, which allows treatment without injuring normal cells and can completely eliminate the vector-infected cells after the therapy is finished, (2) construction of cell-specific expression/replication vector with high safety in the field of gene therapy against fibrosis such as pulmonary fibrosis and hepatic fibrosis, wherein an expression/replication vector that can express genes cell-specifically to specifically disrupt proliferating myofibroblasts is generated, which allows treatment without injuring normal cells and can completely remove the vector-infected cells after the therapy is finished, (3) construction of cell-specific expression/replication vector with high safety in the field of gene therapy for such as vessel constriction, restenosis, diabetic retinopathy and the like after stent placement or organ transplantation, arteriosclerosis and diabetic retinopathy wherein an expression/replication vector that can express genes cell-specifically to specifically disrupt proliferating vascular smooth muscle cells is generated, which allows treatment without injuring normal cells and can completely remove the vector-infected cells after the therapy is finished, (4) construction of cell-specific expression/replication vector with high safety in the field of gene therapy for glomerulonephritis, wherein an expression/replication vector that can express genes cell-specifically to specifically disrupt proliferating mesangial cells is generated, which allows treatment without injuring normal cells and can completely remove the vector-infected cells after the therapy is finished.

### Background Art

Recently, an ideal therapeutic method for cancer with less side effects wherein normal cells are not affected and only the cancer cells can be selectively impaired, has been desired. Gene therapy can be given as one example, and said therapy is capable of increasing the selectivity of the cancer cell at various levels, such as the cell selectivity and expression promoter activity of a gene to be introduced into the cancer cell, or infection and induction method of a viral vector, and has drawn attention as a promising therapy in the future. However, there is a common problem that the therapeutic gene cannot be introduced to all cancer cells. In contrast, in the immunotherapy for cancer, since expression of a tissue-specific differentiation antigen is observed slightly also in normal cells, the side effects to the normal cells have been a problem. Further, since cancer antigen based on mutation has a defect in that the mutation is limited to the individual cancers, it is not suitable to generalize it as an immunotherapy for cancer that is molecule-targeted.

Recently, a clinical study of gene therapy is conducted in the United States and United Kingdom, concerning a malignant brain tumor using a replication-competent herpes simplex virus (HSV) (vector) that continuously and selectively impairs only the proliferating cells by infection and replication (Gene Ther. 7, 859-866, 2000; Gene Ther. 7, 867-874, 2000). Thereplicative HSV vector is a vector wherein Ribonucleotide reductase (RR) or Thymidine kinase (TK) that are essential for viral replication, are deleted. These enzymes are expressed in normal cells only when they are proliferating but expressed constitutively in tumor cells. Therefore, when this HSV vector is infected to a cell that proliferates strongly, regardless of a normal cell or a tumor cell, it replicates with cell-derived RR or TK and shows a cytolytic activity. Meanwhile, in Japan, in an animal experiment, an anti-tumor effect of replicative HSV vectors against prostate cancer and pancreatic cancer has been reported (J. Surg. Oncol. 72, 136-141, 1999), however, these do not have cell selectivity either, and their safety is low. Therefore, it could be used in therapy for human in a brain wherein the vector does not diffuse in the circulating blood due to the presence of a blood brain barrier, however there was a problem in that it was not suitable for treatment in organs aside from the brain.

As explained above, it is considered that it will become a further effective and safer therapy if the impairment activity of the HSV vector can be controlled target cell-specifically. Martuza et al. of the United States have reported of a replication-competent HSV vector that is liver tumor-selective, using an albumin promoter (J. Virol. 71, 5124-5132, 1997). However, when said vector is used in liver cell cancer, the expression of albumin gene decreases and the normal regenerative liver cells are also impaired, and therefore, it is not considered suitable for clinical application in human. The description of U.S. Patent No. 5,728,379 ("Tumor- or cell-specific herpes simplex virus replication") states the possibility of application to mesothelioma, however, it does not state the possibility of application to therapies for human sarcoma in general, such as leiomyosarcoma, osteosarcoma, gastrointestinal stromal tumor (GIST), tumor vessel, proliferating vascular lesion, proliferating glomerulonephritis, fibrosis of lung, liver and the like, or myofibroblast that proliferate at the stroma of malignant tumors.

The existence of fusion gene and mutation of p53 and Rb in some tumors are reported from the genetic analysis regarding the cause of disease and pathology of sarcoma, however, it has not reached the level that can be applied widely to therapies. In an animal experiment using nude mice, Milas et al. used an adenoviral vector without a replication ability to introduce p53 gene into leiomyosarcoma cells, and reported that there was a delayed effect in the proliferation of tumors (Cancer Gene Ther. 7, 422-429, 2000). There is also a report regarding a method for introducing and expressing a suicide gene, thymidine kinase, into osteosarcoma by using an osteocalcin gene promoter (Cancer Gene Ther. 5, 274-280, 1998). However, it uses a viral vector wherein its replication ability is deleted, and the efficiency for gene transfer is poor, therefore, it cannot be applied to sarcoma other than osteosarcoma. Particularly, according to the report by Milas et al., an example using SK-LMS-1, a human smooth muscle cell line is shown, which is the same cell line as the one described in the report (Cancer Res. 61, 3969-3977, 2001) by the present inventors. However, 100 to 1000 fold more amount of viral particles are used in comparison to the amount of particles of the viral vector used in the report mentioned above, and the efficiency is lower than that of the report mentioned above. Therefore, the results of Milas et al. is not preferable, from the viewpoint of suppressing the side effects by minimizing the number of viral particles to be injected into the body.

Further, as a therapy for suppressing angiogenesis of cancer, a dramatic anti-tumor effect of anti-angiogenesis peptides such as angiostatin and endostatin have been reported by an experimental system with mice performed by the group of Folkman in the United States (Cell 79, 315-328, 1994; Cell 88, 277-285, 1997). In Japan, Nakamura et al. have also reported the suppressing action of angiogenesis of NK4, an intramolecular fragment of a hepatocyte growth factor (HGF)(Biochem. Biophys. Res. Commun. 279, 846-852, 2000). However, these methods have problems, such as the requirement for a large amount of peptides, the fact that there is a report that their reproducibility to endostatin is low, the fact that the mechanism is unknown, and further that the efficacy in human has not been confirmed. The inhibitor of angiogenesis, which is currently in clinical trial, does not have cell selectivity and its inhibiting efficiency is low. The peptide which inhibits the action of the integrin on the surface of endothelial cells, reported by Cheresh et al. of the United States, does not have cell selectivity as well, and its inhibiting efficiency is low (J. Clin. Invest. 103, 1227-1230, 1999). These researches all relate to therapies that target vascular endothelial cells, however, cell-selective therapeutic agent targeting tumor vessel composed of proliferating vascular smooth muscle cells has not been known. In fact, it is reported that the antagonist of a platelet-derived growth factor receptor that facilitates the proliferation and migration of smooth muscle cells has a strong suppressing action for tumor angiogenesis (Cancer Res. 60, 4152-4160, 2000), and the importance to attack the vascular smooth muscle in order to suppress the tumor angiogenesis is speculated. However, this method is not cell-selective and side effects are also expected.

Moreover, for the proliferating vascular lesion, in particular, vessel constriction after stent placement and heart transplantation, various agents that suppress the proliferation of smooth muscles of neointima are attempted. However, none of these have succeeded in preventing constriction. As a recent attempt in gene therapy, there is a report by Leiden et al., wherein an adenoviral vector that is deficient in replication ability is used, to selectively introduce a LacZ gene into a smooth muscle cells of a rat carotid artery after balloon injury, under the control of a promoter of SM22a, a homologous gene of calponin (J. Clin. Investi. 100, 1006-1014, 1997). However, in this experiment, it was not the proliferating smooth muscle of the intima, which is a target cell, but the smooth muscle of the tunica media that was introduced with the LacZ gene, and the efficiency of introduction was extremely low. Further, Nabel et al. also conducted an experiment using an adenoviral vector without a replication ability, wherein a LacZ gene, CAT (chloramphenicol acetyltransferase) gene was introduced into pig artery under the control of SM22a prompoter, however, only 2.2% of the intimal smooth muscle cells, 0.56% of the tunica media smooth muscle cells showed gene expression (Mol. Med. 6, 983-991, 2000). In contrast, according to the report by Miyatake et al. wherein a replicative HSV vector was used to infect rat carotid artery after balloon injury (Stroke 30, 2431-2439, 1999), the replication of virus is observed mainly in the proliferating smooth muscles of the initima, and the efficacy of using a replicative viral vector is speculated. However, this virus is not cell-selective and side effects such as the cell disruption of intima cells and adventitial fibroblasts are predicted. Other methods such as directly introducing oligonucleotide such as decoy and antisense DNA into the vessel have also been presented, however, the efficiency of introduction is low and sufficient suppressive effect of vessel smooth muscle proliferation cannot be expected.

Moreover, as an attempt of recent gene therapy regarding proliferating mesangial cells in glomerulonephritis, a method has been reported wherein decorin and TGFB receptor that have TGFB1 inhibiting action and chimeric gene of the IgG Fc region, or decoy of NFkappaB are introduced into the renal glomerulus using a liposome vector (Nature Med. 2, 418-423, 1996; Kidney Int. 55, 465-475, 1999; Gene Ther. 7, 1326-1332, 2000). However, this method is not cell-selective and side effects are also predicted. Moreover, a method has been presented, wherein an adenoviral vector deficient in replication ability is bound to a microsphere of polystyrene and administered to a rat aorta, in order to selectively introduce a gene into a renal glomerulus (Kidney Int. 58, 1500-1510, 2000). However, aside from mesangial cells, which are a cause for proliferating glomerulonephritis, expression of introduced genes is observed also in vascular endothelial cells, and targeting of the therapy remains uncompleted. Further, the immunogenicity of adenovirus is strong, and the high risk for it to evoke the immune response that leads to glomerulonephritis is indicated (Kidney Int. 61, S85-S88, 1997).

Meanwhile, the present inventors have found that a calponin gene, which is thought to be a differentiation marker of smooth muscles, is expressed in the tumor cells of human-derived sarcoma, and reported this fact for the first time (Int. J. Cancer 79, 245-250, 1998; Sarcoma 3, 107-113, 1999; Intern. J. Cancer 82, 678-686, 1999). Thereafter, there have been continuous domestic and foreign reports that calponin genes express abnormally in almost 20 types of human malignant tumor derived from mesenchymal cells such as bone sarcoma and soft tissue sarcoma as well as in gastrointestinal stromal tumor (GIST) and salivary gland sarcoma, fibrosarcoma, malignant neurinoma. It was revealed by analysis of the X-ray crystallographic structure and the in vitro and in vivo functional analyses, that the calponin mentioned above (h1 or basic) binds to the C-terminal region of actin molecules and suppresses the sliding motility of actin and myosin (Biochem. Biophys. Res. Commun. 279, 150-157, 2000; J. Physiol. 529, 811-824, 2000) . In an adult body, the calponin gene selectively expresses in the smooth muscle cell and is regarded as a differentiation marker of the vessels and gastrointestinal tract (Physiol. Rev. 75, 487-517, 1995).

Further, in the description of U.S. Patent No. 5,728,379 mentioned above and the report by the present inventors (Cancer Res. 61, 3969-3977, 2001), it is described regarding a replicative vector deficient in a DNA that encodes a thymidine kinase of HSV. However, HSV deficient in thymidine kinase is not sensitive to aciclovir or ganciclovir, which are anti-herpes virus agents, and when these vectors are applied in therapies for human, there would be serious safety concerns if the expansion of unexpected infection of the virus is ocurred.

Further, a replication-competent HSV-1, G207, wherein both two copies of the gamma 34.5 gene that are involved in the replication in the neuronal cells are deficient, and the LacZ gene is inserted in the ribonucleotide reductase (ICP6)-locus (Nature Med. 1, 938-943, 1995), and a replication-competent HSV-1 vector HSVlyCD wherein an autofluorescent protein and a cytosine deaminase that are expressed by a CMV promoter/enhancer are inserted in the ICP6-locus by homologous recombination (Cancer Res. 61, 5447-5452, 2001) have been known, however, since both are deficient in ribonucleotide reductase, replication with proliferating cells alone is possible but there is no cell selectivity. Moreover, there is no report of a treatment method wherein the proliferating myofibroblast in the fibrosis such as pulmonary fibrosis and hepatic fibrosis is targeted and selectively disrupted. In addition, there have been no report of a treatment method wherein the myofibroblast that proliferate of malignant tumors are targeted.

The object of the present description is to construct a cell-specific expression/repLication vector for use in the therapy for malignant tumors and the like, wherein genes are specifically expressed in a specific cell such as malignant tumor and the like and then replicated, and normal cells are not injured, which can especially suppress the expression and replication at a desired period after the expression and replication. Further, the object of the present description is to provide a treatment method wherein said vector is introduced into the cells of specific organisms such as malignant tumor and the like and then expressed.

The present inventors have made a keen study to elucidate the object mentioned above, and constructed a cell-specific expression/replication vector that does not act in adult normal cells and can induce viral replication by the following steps: obtaining a transcriptional initiation regulatory region in the cells of a human calponin gene that specifically express in specific tumor cells and smooth muscle cells; said region is integrated to the upstream of the gene that encodes the transcription factor necessary to initiate the expression of the viral replication-related gene, thus viral replication; said gene is expressed in specific cells such as malignant tumor cells or proliferating smooth muscle cells of new vessels in the tumors and in the lesions of vascular constriction by substituting this with a TK gene that is an essential enzyme for the replication of viral DNA. It is reported that when the constructed cell-specific expression/replication vector was introduced into a malignant tumor tissue, the tumor cells and the proliferating smooth muscles of the new vessels in the tumors and in the lesions of vascular constriction are selectively impaired (Cancer Res. 61,3969-3977,2001; Japanese Patent Application No. 2001-143999).

The HSV-1 vector that is cell-specifically replicative that have been presented so far are the replication-competent HSV-1 vector having a calponin promoter, which the present inventors have reported (Cancer Res. 61, 3969-3977, 2001; Japanese Patent Application No. 2001-143999) and the replication-competent HSV-1 vector that is liver tumor-selective using an albumin prompter according to Martuza et al. of the United States as mentioned previously (J. Virol. 71, 5124-5132, 1997; U.S. Patent No. 5,728,379). However, their parent strain is an HSV-1 mutant virus d120 wherein both of the two genes that encode ICP4, an essential transcription factor for viral replication are deficient, and LacZ cDNA is linked upstream of the promoter and ICP4 cDNA is linked downstream of the promoter, and thymidine kinase locus (TK-locus) is disrupted by homologous recombination. Therefore, the expression of LacZ gene that serves as an index in the process of vector purification, is under control of the TK gene promoter.

The replication-competent HSV-1 vector wherein the DNA that encodes said thymidine kinase is deficient does not have sensitivity against aciclovir and ganciclovir, which are anti-herpes virus agents. Therefore, the method for purifying the vector to a single clone had been conducted by repeating cycles for plaque purification as follows: a virus mixed solution after homologous recombination was infected to a Vero E5 cell wherein ICP4 cDNA was introduced; multiple, preferably a few plaques were isolated by blue staining which indicates the expression of LacZ genes in a 5-bromo-4-chloro-3-indolyl-B-D-galactopyranoside (X-gal) agarose overlay assay; wherein Vero E5 cells are infected again in the presence of ganciclovir, was repeated. It should be realized that the method for elimination of the virus, wherein recombination at the TK-locus has not occurred, by the anti-herpes virus drug can not be used, that is, the methods for purification used in the virus lacking a TK gene, cannot be applied in the purification of a cell-specific expression/replication vector having a TK gene.

In addition, it is impossible to isolate a single plaque at the initial stage of screening, in the method of X-gal agarose overlay assay. Further, in this method, at the point when the agarose is overlayed, the division and proliferation of the cells stop as well as the replication of the virus, and the amount of viral particles does not increase thereafter. In this method, in the case of a replication-competent vector having a LacZ gene that is expressed by a promoter with an activity stronger than the TK gene promoter, for example, by the promoter of ribonucleotide reductase (RR) gene, if the replication ability of the vector itself is not high and the vector is stained blue in the same level as that of the replication-competent vector having a LacZ gene that is expressed by the promoter of TK gene, the number of virus per cell would be few. For that reason, it is difficult to isolate the vector with replication ability for the next screening.

Further, as a cell-specific expression/replication vector, when an ICP4 cDNA is linked to the optional gene inserted downstream of the IRES (internal ribosomal entry site) (a cDNA that express Green Fluorescent Protein which shows fluorescence can be preferably exemplified), the optional gene mentioned above is expressed under the control of a cell-specific transcriptional initiation regulatory region. Thus, screening using the expression of both the optional gene and the LacZ gene as an index becomes possible, and the present inventors found out that the viral vector wherein homologous recombination is successfully occurred at the desired place can be separated more definitely and rapidly.

Further, the present inventors found out that a vector wherein the objective recombination in that ICP4 is expressed under the control of a cell-specific promoter is occurred can be selected and concentrated by the following method: in the first screening after the homologous recombination, a virus mixed solution after homologous recombination including a cell-specific expression/replication vector is infected to an ICP4 non-expressing cell wherein the promoter of the gene that express cell-specifically, that is, the transcriptional initiation regulatory region, can be activated or is infected to an ICP4 non-expressing cell that expresses said gene wherein the aforementioned virus is replicated and proliferated; then the expression of the gene which is integrated in the vector is made to be an index to purify until a single clone is obtained by limiting dilution. Further, a cell-specific expression/replication vector wherein thymidine kinase is preserved can be inactivated and extinguished with its infected cells by the treatment with aciclovir and the ganciclovir, and the present inventors found out that there is an excellent property in the safety measures of preventing the unexpected expansion of infection of the virus. Meanwhile, the examples of the invention of the U.S. No. 5,728,379, which is a cell-specific replicative HSV-1 vector wherein thymidine kinase is deficient, and of the invention of the previously filed Japanese Patent Application No. 2001-143999 can be considered as not applicable to the therapies for human. Further, the present inventors confirmed by in vitro cell culture system or animal experiment system that the cell-specific expression/replication vector has a therapeutic effect against malignant fibrous histiocytoma (MFH) which actually appears most frequently among the human soft tissue sarcoma, gastrointestinal stromal tumor (GIST) which appears most frequently among the human gastrointestinal sarcoma, and uterine myoma which appears most frequently among the field of gynecology.

The present description relates to: a cell-specific expression/replication vector that does not act to adult normal cells, wherein a transcriptional initiation regulatory region of a gene that expresses cell-specifically is integrated upstream of a predetermined gene, and a thymidine kinase gene that exists in said cell-specific expression/replication vector is used to suppress the replication at a desired period ("1"); the cell-specific expression/replication vector that does not act to adult normal cells according "1", wherein the transcriptional initiation regulatory region of the gene that expresses cell- specifically is a region including the base sequence shown in Seq. ID No. 1 ("2"); the well-specific expression/replication vector that does not act to adult normal cells according to "2", wherein the region including the base sequence shown in Seq. ID No. 1 is a region including a human calponin gene promoter comprising a base sequence shown in Seq. ID No. 2 ("3"); the cell-specific expression/replication vector that does not act to adult normal cells according to "3", wherein the region including a base sequence shown in Seq. ID No. 2 is a region including a base sequence shown in Seq . ID No. 3 ("4"); the cell-specific expression/replication vector that does not act to adult normal cells according to "1", wherein the transcriptional initiation regulatory region of the gene that expresses cell-specifically comprises a base sequence wherein one or a few base is deleted, substituted or added in a base sequence shown in Seq. ID No. 1, Seq. ID No. 2 or Seq. ID No. 3, and is a region including a base sequence having a transcription initiation control activity ("5"); the cell-specific expression/replication vector that does not act to adult normal cells according to any one of "1" to "5", wherein an enhancer is integrated upstream of the transcriptional initiation regulatory region ("6"); the cell-specific expression/replication vector that does not act to adult normal cells according to "6", wherein the enhancer is a 4F2 enhancer ("7"); the cell-specific expression/replication vector that does not act to adult normal cells according to any one "1" to "7" , wherein a DNA that encodes a desired protein different from the predetermined gene is linked further downstream on the predetermined gene, and expresses the desired protein under the control of said transcriptional initiation regulatory region ("8"); the cell-specific expression/replication vector that does not act to adult normal cells according to "8", wherein the DNA that encodes the desired protein is linked to the predetermined gene via an IRES (internal ribosomal entry site) ("9"); the cell-specific expression/replication vector that does not act to adult normal cells according to any one of "1" to "9", wherein the DNA that encodes the desired protein is an apoptosis promotion-related gene ("10"); the cell-specific expression/replication vector that does not act to adult normal cells according to any one of "1" to "9" , wherein the DNA that encodes the desired protein is a DNA that encodes a protein having a suppressive action of angiogenesis ("11"); the cell-specific expression/replication vector that does not act to adult normal cells according to any one of "1" to "9", wherein the DNA that encodes the desired protein is a DNA that encodes a protein having a suppressive action against cancer metastasis ("12"); the cell-specific expression/replication vector that does not act to adult normal cells according to any one of "1" to "9", wherein the DNA that encodes the desired protein is a DNA that encodes a protein having a suppressive action against cancer growth ("13"); the cell-specific expression/replication vector that does not act to adult normal cells according to any one of "1" to "13", wherein the predetermined gene is a viral replication-related gene ("14"); the cell-specific expression/replication vector that does not act to adult normal cells according to "14", wherein the viral replication-related gene is ICP4 or E1A ("15"); the cell-specific expression/replication vector that does not act to adult normal cells according to any one of "1" to "15", wherein the expression/replication vector is a viral vector ("16"); the cell-specific expression/replication vector that does not act to adult normal cells according to "16", wherein the viral vector is a herpes simplex virus vector (HSV vector) or an adenoviral vector ("17"); the cell-specific expression/replication vector that does not act to adult normal cells according to any one of "1" to "15", wherein the vector is tumor cell-specific, proliferating smooth muscle-specific in tumor neovasculature, proliferating smooth muscle-specific in proliferating vascular lesion, proliferating mesangial cell-specific in glomerulonephritis, or proliferating myofibroblast-specific in fibrosis ("18"); and the cell-specific expression/replication vector that does not act to adult normal cells according to any one of "1" to "18", wherein a DNA that encodes ribonucleotide reductase is deleted ("19").

Further, the present description relates to for example only: a method for expression/replication of a gene, protein or a peptide of a cell-specific expression/replication vector that does not act to adult normal cells, wherein the cell-specific expression/replication vector that does not act to adult normal cells according to any one of "1" to "19" is introduced into the cells and tissues of an organism, then expressed and replicated ("20"); a method for suppressing the expression/replication of a gene, protein or a peptide of a cell-specific expression/replication vector that does not act to adult normal cells, wherein the cell-specific expression/replication vector that does not act to adult normal cells according to any one of "1" to "19" is introduced into the cells and tissues of an organism, then expressed and replicated, and the expression/replication of the cell-specific expression/replication vector is suppressed at a later desired period ("21"); the method for suppressing the expression/replication of a gene, protein or a peptide of a cell-specific expression/replication vector that does not act to adult normal cells to "21", wherein the suppression of the cell-specific expression/replication vector is a suppression by using antiviral drugs including aciclovir and ganciclovir ("22"); a method for detecting the in vivo distribution of a cell-specific expression/replication vector that does not act to adult normal cells, wherein the cell-specific expression/replication vector that does not act to adult normal cells according to any one of "1" to "19" is introduced into the cells and tissues of an organism, then expressed and replicated, and the thymidine kinase activity by said cell-specific expression/replication vector is determined ("23"); and the method for detecting the in vivo distribution of a cell-specific expression/replication vector that does not act to adult normal cells according to " 23", wherein the determination of the thymidine kinase activity is a determination by positron emission tomography using an uracil derivative FIAU labeled with ³²⁴I ("24")_{.}

Still further, the present description, relates to the method according to any one of "20" to "24" , wherein the cells and tissues in the organism are tumor tissues, vascular or lymphatic vessel constriction tissues, nephritic tissues or fibrotic tissues (for example only) ("25"); a therapeutic drug comprising the cell-specific expression/replication vector that does not act to adult normal cells according to any one of "1" to "19" ("26"); the therapeutic drug according to "26" , wherein the therapeutic drug is against malignant tumor, fibrosis, proliferating vascular lesion or proliferating glomerulonephritis ("27"); the therapeutic drug according to "27" , wherein the therapeutic drug is against malignant fibrous histiocytoma, gastrointestinal stromal tumor or uterine myoma ("28"); a therapeutic method for fibrosis and malignant tumor, wherein the cell-specific expression/replication vector that does not act to adult normal cells according to any one of "1" to "19" is introduced into fibrotic tissues including lung and liver, or malignant tumor tissues including breast cancer, gastric cancer and pancreatic cancer, then a proliferating myofibroblast is selectively disrupted as a result of replication of a vector, and expression of a gene, protein and a peptide ("29"); the therapeutic method for fibrosis and malignant tumor according to "29", wherein its subject is malignant fibrous histiocytoma, gastrointestinal stromal tumor or uterine myoma ("30"); a therapeutic method for proliferating vascular lesion, wherein the cell-specific expression/replication vector that does not act to adult normal cells according to any one of "1" to "19" is introduced into blood vessel or lymphatic vessel constriction tissues or arteriosclerotic tissues and tissues with diabetic retinopathy, then a proliferating smooth muscle cells or perivascular cells are selectively disrupted as a result of replication of a vector, and expression of a gene, protein or a peptide ("31"); a therapeutic method for proliferating glomerulonephritis, wherein the cell-specific expression/replication vector that does not act to adult normal cells according to any one of "1" to "19" is introduced into a nephritic tissue, then a proliferating mesangial cells are selectively disrupted as a result of replication of a vector, and expression of a gene, protein or a peptide ("32"); the therapeutic method according to any of "29" to "32", wherein the cell-specific expression/replication vector is administered to a vein or artery ("33"); the therapeutic method according to any one of "29" to "33", wherein the expression/replication of the cell-specific expression/replication vector is suppressed at a desired period ( "34" ) ; a method for producing a cell-specific expression/replication vector, wherein a virus mixed solution after homologous recombination including the cell-specific expression/replication vector according to any one of "1" to "19" is infected to a cell, wherein the transcriptional initiation regulatory region of a gene that expresses cell-specifically can be activated or a cell that expresses said gene, and the expression of a gene integrated in the vector is used as an index to purify to a single clone by limiting dilution without using agarose overlay assay ( "35" ) ; and the method for producing the cell-specific expression/replication vector according to "35", wherein the cell is an ICP4 non-expressing cell ("36").

### Brief Description of Drawings

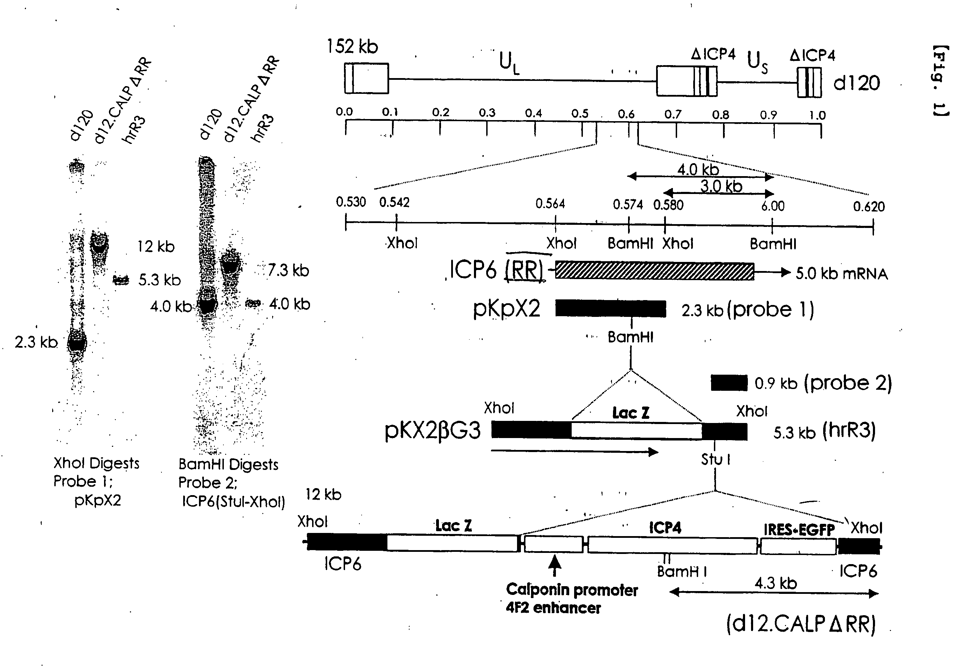

Figure 1 includes a photograph showing the procedure of constructing d12·CALPΔRR and its structure. The view on the left shows the results of Southern blot wherein pKpX2 (XhoI fragment of ICP6) and StuI-XhoI fragment of ICP6 are used as DIG-labeled probes. d120 is a parental strain wherein homologous recombination is conducted, and is a mutant derived from KOS strain wherein both of the two of ICP4 genes are deficient. In hrR3, ICP6 is deleted as a result of introducing a LacZ gene in the BamHI site of the ribonucleotide reductase (ICP6) gene of KOS strain (pKX2BG3), which is a wild-type strain.

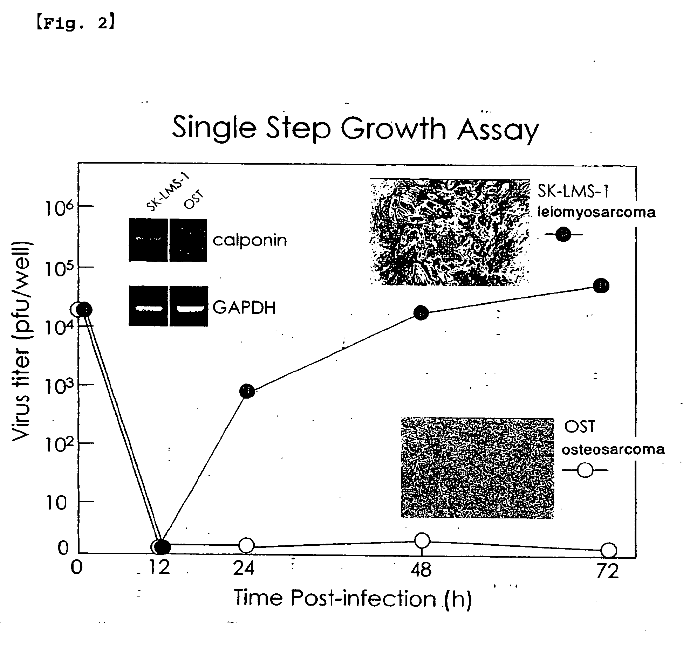

Figure 2 includes a photograph showing the selective cytolytic activity of d12·CALPΔRR against calponin-positive malignant tumor cells (SK-LMS-1 leiomyosarcoma) in vitro. The view on the upper left shows the observation of calponin mRNA expression by RT-PCR, and there is hardly any calponin expressed in OST osteosarcoma cells. The view on the right shows an X-Gal staining of the plaque.

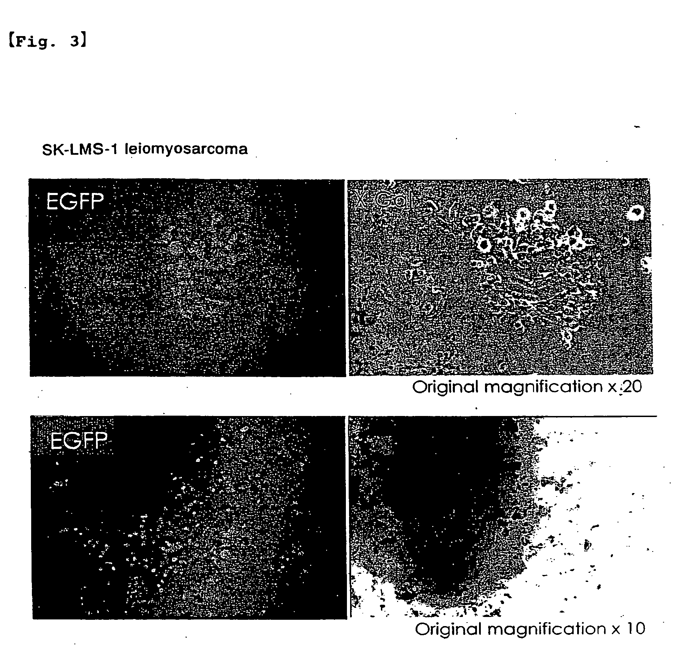

Figure 3 shows the replication of d12·CALPΔRR in the calponin-positive malignant tumor cells (SK-LMS-1 leiomyosarcoma) in vitro with X-Gal staining which indicates the LacZ gene expression, and a photograph of the observation of EGFP protein that expresses under the control of a calponin promoter through a fluorescence microscope. Many cells expressing both LacZ and EGFP can be observed.

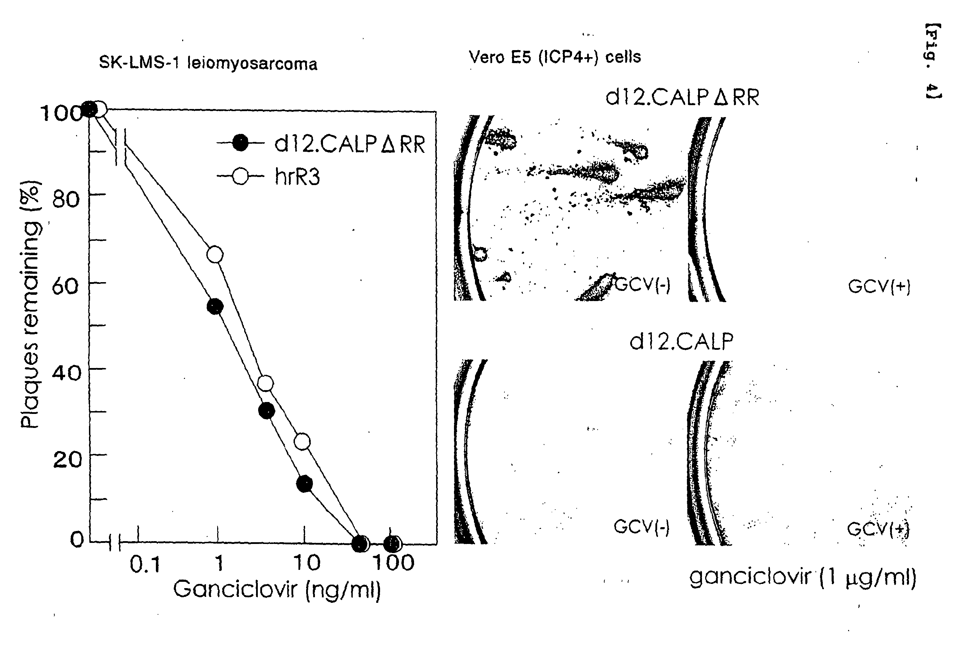

Figure 4 includes a photograph showing the replication of d12·CALPΔRR and the sensitivity of ganciclovir of cytolytic activity in Vero E5 cells introduced with the calponin-positive malignant tumor cells (SK-LMS-1 leiomyosarcoma) and ICP4 cDNA in vitro. The view on the left is a comparison with hrR3, which is known to be hypersensitive to ganciclovir. The view on the right is a comparison with d12·CALP deficient in thymidine kinase (Japanese Patent Application No. 2001-143999), indicating the observation of the cytolytic activity under the presence of 1 µg/ml ganciclovir. d12·CALP is not sensitive to ganciclovir.

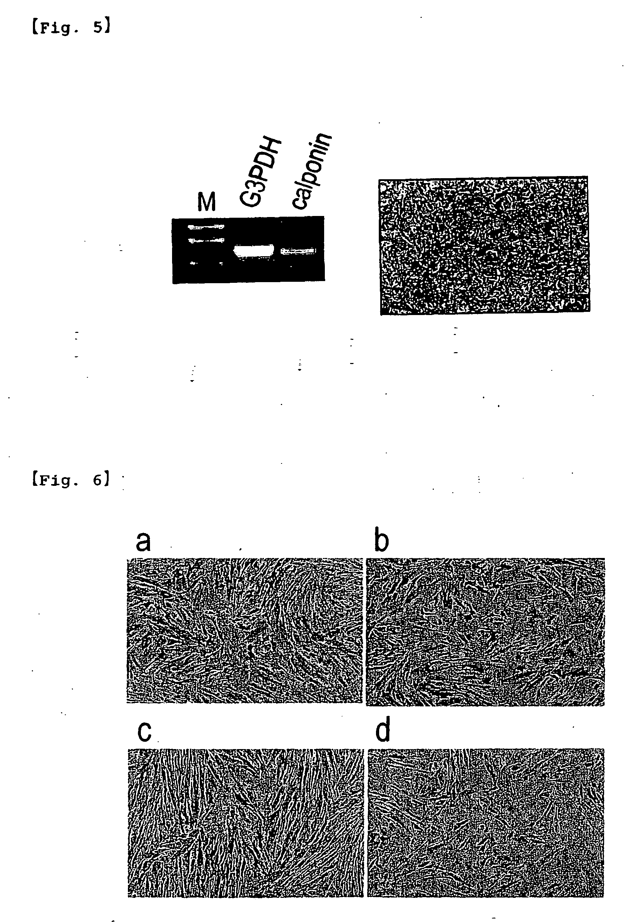

Figure 5 is a photograph showing the expression of calponin mRNA and the cell disruption assay or vector replication assay in vitro. (a) shows the expression of calponin (hl) mRNA in human sarcoma (malignant fibrous histiocytoma). (b) shows the X-Gal staining of plaque when infecting d12·CALPΔRR vector at 0.01 MOI to a tumor.
Figure 6 is a photograph showing the cell disruption assay and vector replication assay in vitro. It shows the X-Gal staining of plaque when infecting a d12·CALPΔRR vector to (a) GIST cells at 0.01 MOI, (b) GIST cells at 0.1 MOI, (c) to uterine myoma cells at 0.01 MOI, (d) to uterine myoma cells at 0.1 MOI, respectively.

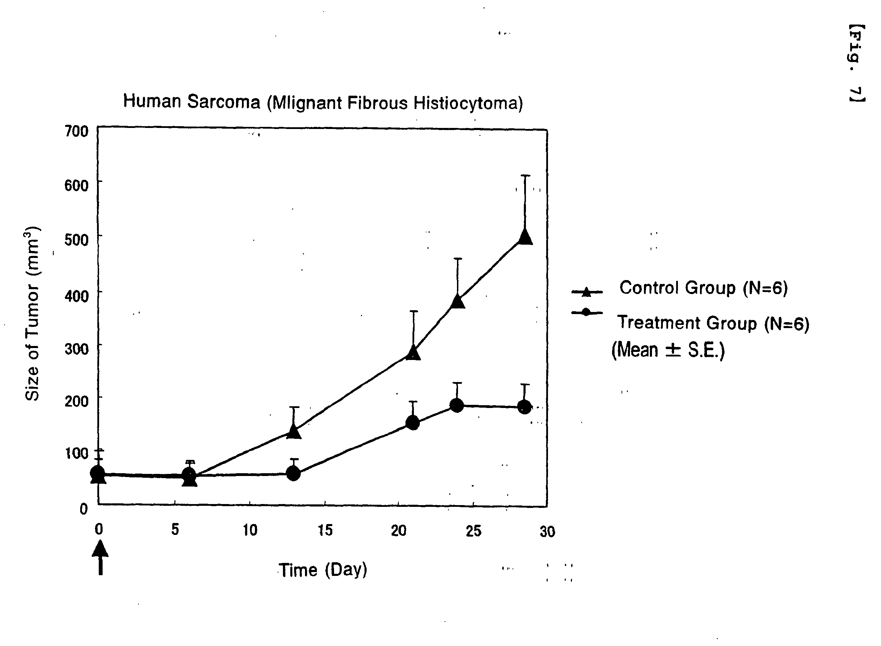

Figure 7 is a graph showing the anti-tumor effect against subcutaneous tumor xenografted in nude mice in vivo.

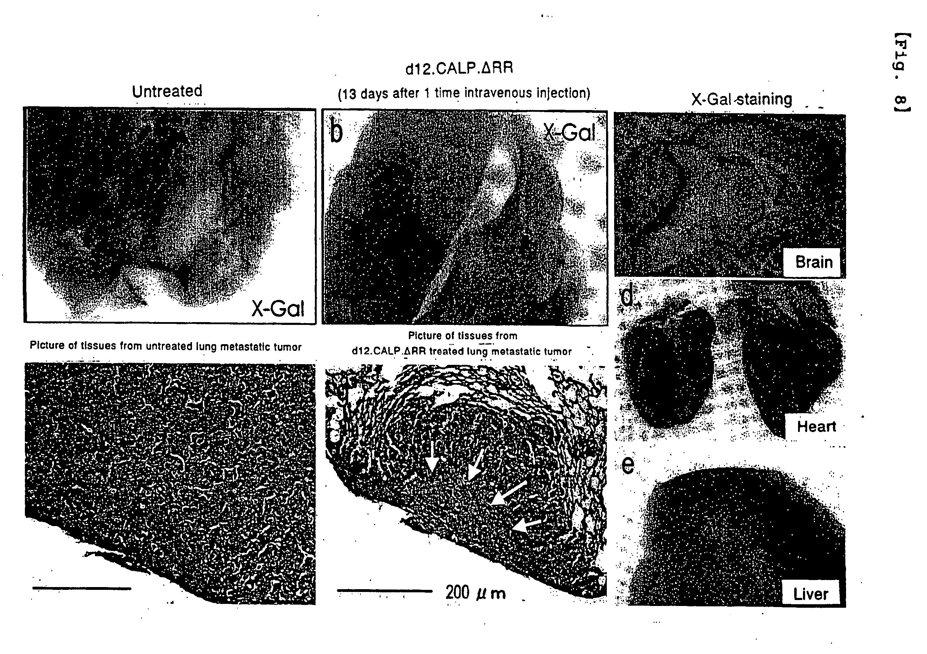

Figure 8 is a photograph showing the analysis of replication and the anti-tumor effect in a lung tumor metastasis in vivo by one intravenous administration of a d12·CALPΔRR vector.

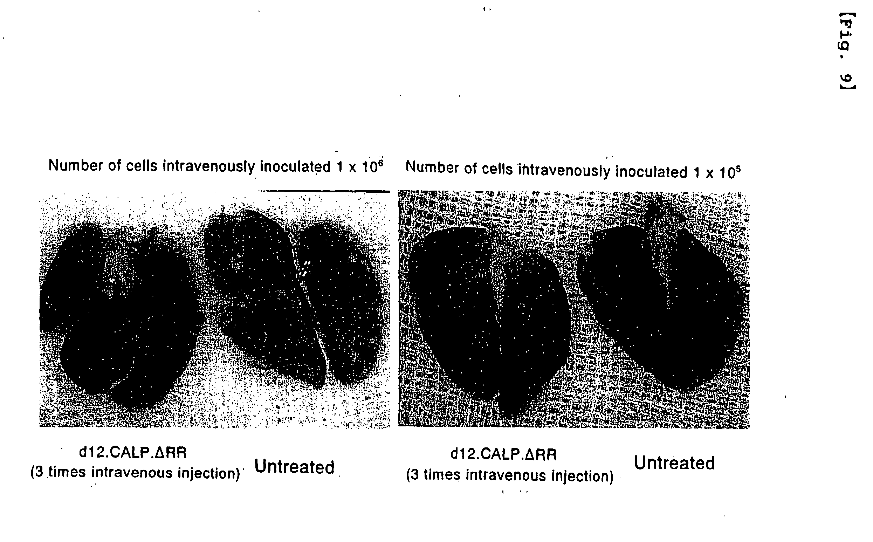

Figure 9 is a photograph showing the therapeutic effect of human lung metastatic tumor by three intravenous administrations of d12·CALPΔRR vector in vivo.

As for the cell-specific expression/replication vector which does not act to adult normal cells of the present description, there is no particular limitation as long as it is a vector which does not act to adult normal cells in which a transcriptional initiation regulatory region of a gene specifically expressed in cells is integrated upstream of a predetermined gene, wherein the thymidine kinase gene present in the cell-specific expression/replication vector is used to suppress its replication at a desired period. However, it is preferable for it to be an expression/replication vector specific to tumor cells, to proliferating smooth muscle cells in tumor angiogenesis, to proliferating smooth muscle cells in proliferating vascular lesion, proliferating mesangial cells in glomerulonephritis, or proliferating myofibroblasts in fibrosis. As a transcriptional initiation regulatory region of the gene specifically expressed in cells mentioned above, a promoter region of a gene specifically expressed in cells or a partial region of said promoter can be exemplified, and more specifically, the examples include: a region including a base sequence from -260 to -219 of a calponin gene promoter shown in Seq. ID No.1; preferably a human calponin gene promoter comprising a base sequence shown in Seq. ID No.2; more preferably a human calponin gene promoter comprising a base sequence shown in Seq. ID No.3 and a region including a part of its structural gene. Furthermore, as for the transcriptional initiation regulatory region of a gene specifically expressed in cells, a base sequence wherein one or a few bases are deleted, substituted or added in the above-mentioned base sequence shown in Seq. ID No.1, Seq. ID No.2 or Seq. ID No.3, having a regulating activity of transcriptional initiation, for example, a region including a homologous region to a calponin promoter derived from mouse, rat and pig can be exemplified.

As for the transcriptional initiation regulatory region of a gene specifically expressed in cells, other than the regions mentioned above, when proliferating smooth muscle cells are targeted for attack, the promoter region of SM22a gene (the sequence from -480 to -26 for human SM22a gene; its homologous region for SM22a gene derived from GenBank accession# D84342-D84344, mouse, rat or other mammals) can be used, and when endothelial cells are targeted for attack, a promoter region of Flk-1 or a promoter region of endothelial cell-specific genes such as Flt-1 gene can be used. In these cases, a region including a part of a structural gene can also be made to be the transcriptional initiation regulatory region.

It is preferable to link an enhancer which significantly activates the transcription at the upstream of a transcriptional initiation regulatory region of a gene specifically expressed in cells mentioned above. As for said enhancer, there is no specific limitation as long as it is an enhancer such as an enhancer of an adenovirus early gene, an enhancer of Moloney murine leukemia virus long terminal repeat, an enhancer of histone H2A gene, an enhancer of immunoglobulin, an enhancer of insulin gene, an enhancer of c-fos gene, an enhancer of T-cell antigen receptor gene, an enhancer of myopathic creatine kinase gene, a transcriptional enhancer of human 4F2 heavy-chain and the like. However, in the case where the transcriptional initiation regulatory region of a gene specifically expressed in cells is a region including a sequence from -260 to +73 of a promoter of a calponin gene, a 4F2 enhancer such as human 4F2 heavy-chain transcriptional enhancer (Seq. ID No.4) which is an enhancer of a 4F2 heavy-chain gene which is a membrane type-II glycoprotein which carry only once the transmenbrane structure which is believed to be an activating actor of an amino acid transporter, is preferable from the point of view that it significantly enhances the transcription efficiency.

As for the predetermined gene to be used for the construction of the cell-specific expression/replication vector that does not act to adult normal cells of the present description, there is no particular limitation as long as it is a gene necessary to initiate or maintain viral replication. For example, viral replication-associated gene such as E1A gene of adenovirus, ICP6 (ribonucleotide reductase) gene and the like can be exemplified, and among them, a gene (ICP 4) that encodes a transcription factor necessary to initiate the replication of herpes virus can be preferably exemplified. Furthermore, as for these genes, it may be a gene wherein a part or whole of the original structural gene located in the downstream of the transcriptional initiation regulatory region is bound with the predetermined gene mentioned above in frame, and a DNA that encodes a fusion protein of a part of the N-terminal side of calponin protein with ICP4 protein can be specifically exemplified.

As for the cell-specific expression/replication vector which does not act to adult normal cells of the present description, a cell-specific expression/replication vector wherein a DNA that encodes a desired protein that is different from the predetermined gene is linked further in the downstream of the predetermined gene, and can express the desired protein under control of said transcriptional initiation regulatory region, is exemplified. Specifically, a cell-specific expression/replication vector wherein the DNA that encodes the desired protein mentioned above is linked to a predetermined gene via IRES (internal ribosomal entry site; description of U.S. Patent No. 4,937,190) can be preferably exemplified. A promoter of SM22a gene, a homologue of calponin, can also be linked to said IRES site. The present inventors are the first to clone a human SM22a promoter sequence and to report it (J. Biochem. (Tokyo) 122, 157-167, 1997), and the base sequence of the portion important for promoter activity (BamH I-DraI fragment 445 bp of human SM22apromoter region) is indicated as Seq. ID No. 5. When CMV promoter and CAG promoter enhancer are used instead of IRES, a gene that goes out of control of calponin promoter and encodes the desired protein can also be expressed in a cell type-nonselective manner.

As for the DNA that encodes the desired protein mentioned above, a gene related to the promotion of apoptosis, a DNA that encodes a protein having an action to suppress neoangiogenesis, a DNA that encodes a protein having an action to suppress cancer metastasis, a DNA that encodes a protein having an action to suppress cancer and the like, can be given as specific examples, and more than two among these DNAs may be linked. Specific examples of the gene related to the promotion of apoptosis mentioned above include: apoptosis-promoting gene such as Bcl-xs, Bok/Mtd, Bcl-Gs/Bra, Bcl-GL, Bcl-Rambo, Hrk/DP5, Bik/Nbk/Blk, Bad, Bid, BimL, S, EL/BodL, M, S, Noxa/APR, Puma and the like; specific examples of the DNA that encodes a protein having an action to suppress neoangiogenesis include: DNA that encodes dominant negative receptor proteins such as angiostatin, endostatin, soluble Flk-1, soluble Flt-1, soluble FLT4, Tiel, Tie2 and the like; specific examples for the DNA that encodes a protein having an action to suppress cancer metastasis include: a DNA that encodes a protein such as matrix metalloprotease (MMP) inhibitor, bovine lactoferrin (bLF) and the like; specific examples of the DNA that encodes a protein having an action to suppress cancer include a DNA that encodes cell cycle suppressor such as p21, p16 , p15 and the like or cell proliferation suppressor such as p53, Rb, IRF-1, APC and the like, however, they are not limited to these examples.

As for the DNA that encodes the desired protein mentioned above, a gene that encodes a marker protein such as EGFP cDNA and luciferase gene are exemplified, and a cell-specific expression/replication vector that can express these marker proteins is significantly useful in screening, detection of the expression/replication vector and the like.

As for the backbone of the viral vector used for the construction of the cell-specific expression/replication vector which does not act to normal cells in adult body of the present description, it is preferable for it to be a vector that can be expressed by being infected or introducing the gene to such as osteosarcoma or soft tissue sarcoma, including leiomyosarcoma, gastrointestinal stromal tumor (GIST), malignant mesothelioma, malignant fibrous histiocytoma (MFH), fibrosarcoma, malignant meningioma, uterine myoma, neurinoma and the like, or proliferating smooth muscle cells or perivascular cells of tumor neovasculature. As for said vector, an expression vector derived from chromosome, episome, liposome and virus can be exemplified. However, viral vector including papovavirus such as SV40, vaccinia virus, adenovirus, adeno-associated viral vector, fowl pox virus, pseudorabies virus, vector derived from retrovirus, herpes simplex virus vector (HSV vector) and the like are preferable, and among these, HSV vector and adenovirus vector, especially a conditionally replication-competent HSV vector or a conditionally replication-competent adenoviral vector is preferable from the viewpoint of the high efficiency of gene expression, the cytotoxic activity Specific to proliferating cells, or the like. By using, for example, a vector wherein the DNA that encodes ribonucleotide reductase is deleted as the conditionally replication-competent HSV vector mentioned above, the cell-specific expression/replication vector which does not act to adult normal cells and can control the replication of the vector and expression of the gene of the present description can be preferably constructed.

As for the method for expression/replication of the cell-specific expression/replication vector that does not act to adult normal cells of the present invention, there is no particular limitation as long as it is a method for expression/replication wherein the cell-specific expression/replication vector that does not act to adult normal cells mentioned above is directly introduced into cells and tissues of an organism, preferably a tissue wherein tumors such as bone/soft part sarcoma, leiomyosarcoma, gastrointestinal stromal tumor, malignant mesothelioma, malignant fibrous histiocytoma, fibrosarcoma, malignant meningioma, neurinoma and the like are developed, or vessel constriction or arterial constriction after stent placement or organ transplantation, nephritic tissue, fibrosis tissue, or an organ including these tissues, or injected from the vascular system that nourishes the tumor, or directly injected using a stent or the like into the vessel. When the proliferating smooth muscles of the new tumor blood vessels are targeted for attack, a direct introduction or injection from the vascular system that nourishes the tumor, whatever the type of malignant solid tumor, can be exemplified. Further, as a method for expression/replication or suppression of a gene, a protein or a peptide of the cell-specific expression/replication vector that does not act to adult normal cells of the present invention, there is no particular limitation as long as it is a method for suppressing the expression/replication of the cell-specific expression/replication vector, wherein the cell-specific expression/replication vector that does not act to adult normal cells mentioned above is expressed/replicated by introduction into the cells and tissues of an organism mentioned above, and an antiviral drug such as aciclovir, ganciclovir and the like, for example, are used later at a desired period. Furthermore, as for the therapeutic agent of the present description, any kind of agent may be used as long as it comprises the cell-specific expression/replication vector that does not act to adult normal cells of the present description mentioned above as an active ingredient. Specific examples of said therapeutic drug include a therapeutic agent against cells and tissues of an organism, preferably the malignant tumors mentioned above, fibrosis, proliferating vascular lesion, proliferating glomerulonephritis, and the like.

As for the therapeutic method for fibrosis and malignant tumor of the present description (included for example only), there is no particular limitation as long as it is a method wherein the cell-specific expression/replication vector that does not act to adult normal cells of the present description is introduced into fibrotic tissues including pulmonary fibrosis and hepatic fibrosis and malignant tumor tissues including breast cancer, gastric cancer and pancreatic cancer, then a gene, a protein or a peptide is expressed. Particularly, a method wherein only the proliferating myofibroblast is selectively disrupted, or a method wherein only the proliferating smooth muscle cells of tumor neovasculature or perivascular cells are selectively disrupted, is preferable. As for the method for introduction into the tissues wherein malignant tumor is developed, a method for directly injecting the cell-specific expression/replication vector mentioned above into the malignant tumor, or a method for injecting the cell-specific expression/replication vector into the tumor via perfusing vascular system, such as arterial or venous administration and the like, can be preferably exemplified. As for the therapeutic method for the proliferating vascular lesion of the present description (included for example only) there is no particular limitation as long as it is a method wherein the cell-specific expression/replication vector that does not act to adult normal cells of the present invention mentioned above, is introduced into a lesion of vessel constriction or arteriosclerotic tissues and diabetic retinopathy tissues, then a gene, protein or peptide is expressed. A method wherein only the proliferating smooth muscle cells or perivascular cells are selectively disrupted can be preferably exemplified. Further, as to the method for treating proliferating glomerulonephritis of the present description, there is no particular limitation as long as it is a method wherein the cell-specific expression/replication vector that does not act to adult normal cells of the present description mentioned above is introduced into a lesion of glomerulonephritis, then a gene, a protein or a peptide is expressed, and among them, a method wherein only the proliferating mesangial cells are selectively disrupted can be preferably exemplified. Moreover, in the therapeutic methods mentioned above of the present description, and included for example only, they are most importantly characterized in that the expression/replication of the cell-specific expression/replication vector is suppressed at a desired period, such as after the completion of the therapy.

As for the method for detecting the in vivo distribution of the cell-specific expression/replication vector that does not act to adult normal cells of the present description, the method is characterized in that the cell-specific expression/replication vector that does not act to adult normal cells of the present description mentioned above is introduced into the cells and tissues of an organism, then expressed/replicated to detect/determine the thymidine kinase activity by said cell-specific expression/replication vector. Specifically, the in vivo distribution of the cell-specific expression/replication vector can be detected by administering an uracil derivative FIAU labeled with ¹²⁴I into the organism, and detecting/determining the ¹²⁴I by Positron Emission Tomography (Nature Med. 7, 859-863, 2001).

As for the method for producing the cell-specific expression/replication vector of the present description, there is no particular limitation as long as it is a screening method wherein a virus mixed solution after homologous recombination including the cell-specific expression/replication vector that does not act to adult normal cells of the present description mentioned above is infected to a cell of which the transcriptional initiation regulatory region of a gene that expresses cell-specifically can be activated or a cell that expresses said gene, preferably an ICP4 non-expressing cell, and the expression of a gene integrated in the vector is used as an index to purify to a single clone by limiting dilution. With the establishment of the method for producing the cell-specific expression/replication vector of the present description conducted by screening, the cell-specific expression/replication vector that does not act to adult normal cells of the present description mentioned above can be obtained for the first time.

Yamamura, H, et al. (Cancer Research 61, 3969-3977, 2001) describe a conditionally replicating HSV vector in which the calponin promoter drives expression of ICP4.

WO 021092816 discloses a similar vector to Yamamura, wherein the transcriptional initiation regulatory domain of human calponin gene, which is specifically expressed in cells, is obtained and ligated to the upstream of a virus replication-associated gene. Then the ligated product is integrated into a virus DNA to thereby construct a replication vector showing cell-specific expression which would not act on normal cells in an adult. The thus constructed vector is transferred into malignant tumor cells to thereby selectively injury tumor cells or proliferative smooth muscle cells in tumor neovessels.

Mineta et. al (Nature Medicine Vol. 1, No. 9, 938-943, 1995) discloses a double mutant HSV (G207) for treating human malignant brain tumours which is replication-competent in dividing cells. This mutant is hypersentitive to ganciclovir and contains a lacZ gene inserted into the ICP6 gene.

Matuza, R. L. (J. Clinical Investigation, Vol. 105, No. 7, 841-846, 2000) discusses the use of genetically engineered viral vectors for tumour therapy, wherein the vectors derive from HSV. A vector containing a mutation in the U*_{L}*39 gene, which encodes the large subunit of HSV ribonucleotide reductase is also disclosed.

Hunter et. al. (J. Virol, Vol. 73, No. 8, 6319-6326, 1999) studied the safety of intracerebral inoculation of the G0207 HSV recombinant in non-human primates.

### Disclosure of the Invention

The present invention relates to a herpes simplex virus (HSV) vector which induces viral gene expression and viral replication specifically in a cell expressing calponin and proliferating, which is not replicated in adult normal cells, and which is capable of suppressing its replication at a desired timing by using the thymidine kinase gene, wherein the vector comprises a DNA fragment comprising:
(i) a promoter region of the human calponin gene comprising the nucleotide sequence shown by SEQ ID No: 3;
(ii) the ICP4 gene encoding a transcription factor essential for initiation of a herpes viral replication which is integrated downstream of the promoter region of the human calponin gene;
(iii) the EGFP gene linked to the downstream of the ICP4 gene via an internal ribosomal entry site; and
(iv) the LacZ gene which is integrated upstream of said promoter region of the human calponin gene
and wherein this DNA fragment is inserted by recombination into the ribonucleotide reductase gene focus of a HSV vector that comprises an endogenous thymidine kinase gene and lacks function of the endogenous ICP4 gene,
and wherein the expression of both the LacZ gene and the EGFP gene integrated in the vector are used as markers to identify the recombinant HSV vector.

The present invention also relates to the HSV vector according to the above, wherein an enhancer is integrated upstream of the promoter region of the human calponin gene.

The present invention also relates to the HSV vector according to the above, wherein the enhancer is a 4F2 enhancer.

The present invention also relates to the therapeutic drug comprising the HSV vector described above.

The present invention also relates to the a method for producing an HSV vector as described above which vector induces viral gene expression and viral replication specifically in a cell expressing calponin and proliferating, which is not replicated in adult normal cells, and which is capable of suppressing its replication at a desired timing by using the thymidine kinase gene, the method comprising the steps of:
(a) preparing a DNA fragment comprising,
   (i) a promoter region of the human calponin gene comprising the nucleotide sequence shown by SEQ ID No. 3,
   (ii) the ICP4 gene encoding a transcription factor essential for initiation of a herpes viral replication which is integrated downstream of the promoter region of the human calponin gene,
   (iii) the EGFP gene linked to the downstream of the ICP4 gene via an internal ribosomal entry site, and
   (iv) the LacZ gene integrated upstream the promoter region of the human calponin gene;
(b) preparing recombinants by cotransfection with the HSV vector that comprises an endogenous thymidine kinase gene and lacks function of the endogenous ICP4 gene together with the DNA fragment into a cell in which a promoter region of the human calponin gene that comprises the nucleotide sequence shown by SEQ ID No: 3 can be activated or a cell which expresses the human calponin gene; and
(c) in order to screen a recombinant HSV vector wherein the DNA fragment is inserted by a homologous recombination into the ribonucleotide reductase gene focus of the HSV vector that comprises an endogenous thymidine kinase gene and lacks function of the endogenous ICP4 gene from among the recombinants, selecting a single clone of the HSV vector from the recombinants by limiting dilution without using agarose overlay assay using the expressions of both the LacZ gene and the EGFP gene as markers.

The present invention will be explained with the following examples.

### Example A [Methods and materials]

### A-1 (Cells, culture methods, antibodies and viruses)

Human uterine leiomyosarcoma cell line SK-LMS-1 (HTB-88) and Vero cells (CCL-81) were purchased from American Type Culture Collection. Human osteosarcoma cell line OST (RCB0454) was purchased from RIKEN GENE BANK. For Vero E5 cells, the Vero cells wherein the ICP4 gene is transfected, those provided by N. Deluca (University of Pittsburgh School of Medicine, Pittsburgh) were used. For human malignant fibrous histiocytoma cell line (MFH-AI), those provided by Dr. Yanoma of Kanagawa Prefectural Cancer Center were used. As for the human gastrointestinal stromal tumor (GIST) cells and the human uterine myoma cells, tumor foci were aseptically prepared from the surgery specimen wherein the expression of calponin protein was confirmed by immunohistochemistry, treated with collagenase (1 mg/ml; Sigma Cat. # C-9722) solution, and the primary culture cells were separated. Those for vector infection experiment wherein 3 to 4 generations of subculture was conducted in an RPMI1640 medium were used. SK-LMS-1 was cultured in Eagle's MEM supplemented with 1 mM sodium pyruvate. OST, Vero cells and Vero E5 cells were cultured in DMEM. MFH-AI was cultured in RPMI1640 medium. All the media contain the following, respectively: heat-inactivated fetal bovine serum (Upstate Biotechnologies) at a final concentration of 10%; 2 mM L-glutamine; 100 units/mL penicillin; and 100 µg/mL streptomycin. Furthermore, all the cells mentioned above were cultured at 37° C in a humidified atmosphere containing 5% CO_{2.}

The MFH-AI cells mentioned above were subcutaneously injected into the franks of six-week old female athymic nude mice (BALB/c Slc-nu/nu) (Japan SLC), and the tumors were fixed. The mice were dissected after two months, the tumor section that was aseptically excised from the metastasized foci in the lung, then treated with collagenase (1 mg/ml; Sigma Cat. # C-9722) and the cells were separated. The 1 × 10⁶ cells were injected into the tail vein of the six-week old female athymic nude mice. One month later, the individual tumor cells were separated from the tumor foci that metastasized again to the lung, in the same manner as described previously. This operation was repeated one more time, and the MFH-AI-LM cell line with high metastatic activity to lung was established.

The monoclonal antibody to HSV-1 or HSV-2 ICP4 protein (clone No. 1101) was purchased from Goodwin Institute for Cancer Research. Immunoblot analysis was carried out in the same manner as described previously (Int. J. Cancer 79, 245-250, 1998). Chemiluminescence (ECL; Amersham Pharmacia Biotech) was used to visualize the bound antibodies, according to the manufacturer's protocol. Moreover, ICP4 deficient mutant HSV-1 d120 (J. Virol 56, 558-570, 1985) and ICP6 (ribonucleotide reductase) -deficient mutant HSV-1 hrR3, which were generated by low-multiplicity infections to ICP4-introduced Vero E5 cells or Vero cells, respectively, were kindly provided by Drs. N. Deluca and S. Weller (University of Connecticut Health Center, Farmington).

### A-2 (RNA preparation and RT-PCR analysis)

Total RNA was extracted from cultured cells or tissues using the Isogene RNA extraction kit (Nippon Gene), and subjected to semi-quantitative RT-PCR analysis as described previously (Int. J. Cancer 79, 245-250, 1998). As to the condition for PCR amplification, a cycle of denaturation at 94° C for 40 seconds, annealing at 60° C for 30 seconds and extension reaction at 72°C for 90 seconds was repeated 30 times. As a human calponin primer, 5'-gagtgtgcagacggaacttcagcc-3' [forward primer 1 (FP1) ; nt # 10-33 GenBank D17408; Seq. ID No. 6] and 5'-gtctgtgcccagcttggggtc-3'[reverse primer 1 (RP1): nt # 660-680; Seq. ID No. 7] were used; as a primer of GAPDH (glyceraldehyde 3-phosphate dehydrogenase) as a control, 5'-cccatcaccatcttccagga-3' [forward primer 2 (FP2); nt# 342-360; Seq. ID No. 8] and 5'-ttgtcataccaggaaatgagc-3' [reverse primer 2 (RP2); nt # 1052-1070; Seq. ID No. 9 were used, to amplify the DNA fragments of 671bp and 731bp, respectively.

### A-3 (Isolation of the human calponin promoter)

Genomic clones containing the 5' upstream region of the human calponin gene were isolated by screening a human genomic λEMBL3 phage library according to the method as described previously (J. Biochem. 120, 18-21, 1996). The 5'-deleted fragments, p-1159Luc, p-385Luc, p-343Luc, p-310Luc, p-299Luc, p-288Luc, p-260Luc, p-239Luc, p-219Luc, p-201Luc, p-176Luc, p-153Luc were generated by PCR amplification, with the genomic clone as a template. Numbers indicate the 5'end of the DNA fragments upstream from the ATG translational initiation codon, hereinafter referred to as +1. These deleted fragments have a common 3' end at position +73. The nucleotide sequence of the cloned fragments was determined by using a DQS-2000L DNA sequencer (SHIMADZU) according to the manufacturer's protocol, and it was confirmed that the sequence was identical to the sequence (DDBJ/GenBank^{™}/EMBL database; accession No. D85611) as described previously (J. Biochem. 120, 18-21, 1996). The minimum expression regulation region (-260 to +73) was identified by the method as described previously (Cancer. Res. 61, 3969-3977, 2001).

### A-4 (Transfection and luciferase assay)

Cells cultured beforehand were divided and were plated onto a plate 24 hours before transfection. Cells (5 × 10⁴) were transfected by injecting 1.2 pg of the promoter plasmid, 0.3 pg of the pCAGGS/B-gal-containing plasmid and 3.75 µl of FuGENE^{™}6 transfection reagent (Roche) in each well of a 6-well dish, according to the manufacturer's protocol. Twenty-four hours after transfection, the cells were harvested in 100 µl/well of the cell lysis buffer (PicaGene^{™} Luciferase Assay System, Toyo Ink). After centrifugation at 4°C at 12000 g for 5 minutes, the supernatants (20 µl or 30 µl) were used for luciferase assay and B-galactosidase assay, respectively. Luciferase activity was measured by using a BLR-201 luminescence reader (Aloka). B-galactosidase assay was carried out by using B-galactosidase enzyme assay system (Promega) following the method as described previously (J. Biochem. (Tokyo) 122, 157-167, 1997). All experiments were repeated at least three times to confirm the reproducibility. By assaying B-galactosidase activity of the cell extracts, the transfection efficiency was determined, and luciferase activities (light units) were corrected according to the value. By comparing expression of the pSV2-Luc gene containing the SV40 enhancer and SV40 promoter, transfection efficiency of different cell lines was evaluated. Data are expressed as % for normalized absorbance ± S.E. relative to the values of pSV2-Luc.

### A-5 (Virus preparation)

A 4.1 kb blunt-ended SalI-MseI fragment (provided by Dr. Hayward, Johns Hopkins School of Medicine) derived from pGH108 (J. Virol. 56, 558-570, 1985) containing ICP4 coding region, was inserted into the blunt-ended BamHI site downstream of the 333 bp human calponin promoter (-260 to +73) cloned to the pAMP1 plasmid, and a 444 bp NotI fragment of the human 4F2 heavy-chain transcriptional enhancer (Mol. Cell Biol. 9, 2588-2597, 1989) (provided by Dr. Leiden, Harvard Medical School) was subcloned to the SmaI site of said plasmid. The HindIII site at the 3' side of pAMP1/CALP-ICP4 plasmid was blunted, the pIRES2-EGFP plasmid (Clontech) was double digested with BamHI and AflII, and the resulting 1576-bp fragment was subcloned. This BamHI-AflII fragment is composed of IRES sequence (description of U.S. Patent No. 4,937,190) and EGFP sequence (description of U.S. Patent Nos. 5,625,048 and 5,804,387) as well as the SV40 derived poly A signal. Further, the 6.7-kb fragment obtained by double digestion of pAMP1/CALP-ICP4-IRES2-EGFP plasmid with the use of EcoRI and SphI was blunted, and was subcloned into StuI blunted site of pKX2BG3 recombinant vector (pKX2BG3/CALP-ICP4-IRES2-EGFP). The pKX2BG3 recombinant vector (provided by Dr. Weller of the University of Connecticut) is comprised of a 2.3-kb XhoI fragment of the ICP6 coding sequence (pKpX2) in the pUC19 backbone, and the 3.0-kb Eschericia coli LacZ sequence is inserted into the BamH1 site of the ICP6 sequence (J. Virol. 62, 196-205, 1988).

Subsequently, the plasmid pKX2BG3/CALP-ICP4-IRES2-EGFP was linearized at XhoI site (one wherein the XbaI site at the 5' side of the ICP6 sequence of pKX2BG3 and the HindIII site at the 3' side of the ICP6 sequence are both substituted by XhoI site), and the pRRΔ-CALP-ICP4-IRES2-EGFP wherein the pUC19 sequence is eliminated and the d120 virus DNA were co-transfected to a subconfluent monolayer culture of ICP4 cDNA-transfected Vero E5 cells (2.5 × 10⁵/well) in a 6-well tissue culture plate, by using Lipofectamine^{™} (GIBCO/BRL), according to the manufacturer's protocol. Three hours after the transfection, 1 ml of 20% FBS/DMEM culture solution was added, and the resultant transfected cells was cultured in said culture solution (10% FBS/DMEM) containing 0.5 mg/ml of 4-hydroxymethylbenzoic acid (HMBA) for 96 hours after the transfection. After confirmation of plaque formation, culture was further conducted for 24 hours with 10% of FBS/DMEM without HMBA. The cells were suspended in 500 pl/well of cold virus buffer (20 mM Tris-HCl containing 150 mM of NaCl; pH 7.5) and then frozen for conservation.

Freezing and thawing treatment with the combination of sonication (30 seconds for 3 times) were conducted three times, and the suspended cells in the solution mentioned above were lysed. The suspended cell solution was diluted stepwisely and infected to the subconfluent monolayer culture of SK-LMS-1 cells in a 96-well tissue culture plate. After the infection, culture was conducted for 96 hours in 100 pl/well of 1% FBS/DMEM containing 11.3 µg/ml of human IgG (Jackson ImmunoResearch Lab.). The wells wherein plaque formation was confirmed were screened with the expression of EGFP under a fluorescence microscope as an index. The SK-LMS-1 monolayer culture cells of the well containing EGFP positive-plaques were suspended in 100 µl of said culture solution, and 6 µl among them were used to determine the B-galactosidase enzyme activity with 5-bromo-4-chloro-3- indolyl-B-D-galactopyranoside (X-gal) as a substrate, by using a B-galactosidase enzyme assay system (Promega). The SK-LMS-1 cells suspended solution of a well that is positive for B-galactosidase enzyme activity was centrifuged for 5 minutes at 5000 rpm, and the pellet was re-suspended in 100 µl/well of cold virus buffer. Limiting dilution, infection and B-galactosidase enzyme activity determination conducted in the same manner using a 96-well tissue culture plate were repeated two times with the Vero E5 cells, and a recombinant viral vector d12·CALP·ΔRR was purified as a single plaque. After purification of the viral DNA, it was digested with the restriction enzyme XhoI, and recombination at the ribonucleotide reductase locus (ICP6 or RR-locus) was confirmed by Southern blotting with the XhoI fragment (2.3-kb) of ICP6 cDNA as a probe (Figure 1).

Viruses were prepared by infecting to Vero E5 cells in 10 to 20 bottles of 150cm²/tissue culture flasks (IWAKI CLASS) and retrieving cells that were detached after 48 hours. Cells were collected by centrifugation at 4°C for 5 minutes at 400 × g, then suspended in 10 ml cold virus buffer (20 mM Tris-HCl containing 150 mM NaCl; pH 7.5). Freezing and thawing treatment with the combination of sonication (30 seconds for 3 times) were conducted three times, and the cells mentioned above were lysed. After centrifugation at 4°C for 5 minutes at 1500 × g, the supernatant was further centrifuged at 4°C for 45 minutes at 15000 × g. The resulting pellet was re-suspended in the cold virus buffer, and titers of the purified d12·CALP·ΔRR viral vector were determined by the plaque assay in Vero E5 cells.

### A-6 (In vitro cytolysis assay and single step growth assay)

The d12·CALP·ΔRR viral vector was infected to subconfluent monolayer culture of cells in a 6-well tissue culture plate at a multiplicity of infection (MOI) of 0.1 to 0.001 (pfu/cell) in 1% heat inactivated FBS/PBS. Said infected cells were incubated at 37°C for 1 hour, and then cultured in said medium containing 1% FBS and 11.3 µg/ml of human IgG (Jackson ImmunoResearch Lab.). Forty-eight hours after the infection, numbers of plaques/well were counted. For single step growth assay, monolayer cultures of SK-LMS-1 cells or OST cells in 12-well tissue culture plates (2 × 10⁵ cells/well) were infected with d12·CALP·ΔRR viral vector to a multiplicity of infection (MOI) of 0.1 in 1% FBS/PBS. The virus inoculum was removed after 1 hour, and the above-mentioned cells were incubated in said medium. The infected cells were harvested from the wells at the predetermined period (12 hours, 24 hours and 48 hours) with the use of 100 µl of the virus buffer. The cell suspension (1 µl) were diluted to 10⁻³, 10⁻⁴ and 10⁻⁵, and then plaque forming activity of viruses on Vero E5 cells were determined.

Further, the d12·CALP·ΔRR viral vector was infected to subconfluent monolayer culture of MFH-AI-LM cells (cell lines with high metastatic activity to lung from human malignant fibrious histiocytoma MFH-AI cells) in a 6-well tissue culture plate at a multiplicity of infection (MOI) of 0.01/cell in 1% heat inactivated FBS/PBS. In addition, the d12·CALP·ΔRR viral vector was infected to subconfluent monolayer culture of human GIST cells and cultured human uterine myoma cells in a 6-well tissue culture plate at a multiplicity of infection (MOI) of 0.1 or 0.01 (pfu/cell), respectively. Said infected cells were incubated at 37°C for 1 hour, and then cultured in said medium containing 1% FBS and 11.3 µg/ml of human IgG (Jackson ImmunoResearch Lab.). Seventy-two hours after the infection, X-Gal staining was conducted and the numbers of plaques/well were counted.

### A-7 (Analysis of sensitivity against ganciclovir, an anti-herpes virus agent of viral replication in vitro)

Virus was infected to the subconfluent monolayer culture of SK-LMS-1 cells in a 24-well tissue culture plate (5 × 10⁴/well) or a 6-well tissue culture plate (2.5 × 10⁵/well) at a multiplicity of infection (MOI) of 0. 01 (pfu/cell) in 1% heat inactivated FBS/PBS. Said infected cells were incubated at 37°C for 1 hour, and then cultured in said medium containing 1% FBS and 11.3 µg/ml of human IgG (Jackson ImmunoResearch Lab. ) , and various concentrations (0 to 1 µg/ml) of ganciclovir (Wako Pure Chemical Industries, Ltd.). Forty-eight hours after the infection, numbers of plaques/well were counted.

For immunoblot analysis of ICP4 expression, d12·CALP·ΔRR viral vector or virus buffer alone was infected to the SK-LMS-1 cells and OST cells, respectively, to a multiplicity of infection (MOI) of 0.01 (pfu/cell), and was isolated after culture for 22 hours. The same amount of protein was subjected to 9% SDS-PAGE gel electrophoresis, and transferred to a nitrocellulose membrane (Bio-Rad). 5% skim milk (DIFCO Laboratories) was used to block the membrane at room temperature for two hours, and incubation was conducted overnight at 4°C by using a said anti-ICP4 antibody (dilution rate 1:10000)

### A-8 (Treatment in vivo and histological analysis)

In order to study the therapeutic effect of one intravenous administration of d12.CALP·ΔRR viral vector against human subdermally transplanted tumor xenografts, 1 × 10⁷ human malignant fibrous histiocytoma MFH-AI cells were subcutaneously injected to the flank of six-week old female athymic nude mice (BALB/c Slc-nu/nu) (Japan SLC) , and the tumors were fixed. After 19 days from the transplantation to the nude mice, the tumors developed from a diameter of approximately 6 mm to 7 mm (50 to 70 mm³) . 100 µl of virus suspension containing 1 × 10⁷ pfu/mouse of d12.CALP·ΔRR viral vector (n = 6) , or the same amount of virus buffer (n = 6) were injected into the tail vein once, respectively, by using a 30 gauge needle. The tumors were measured at a predetermined period after the injection, and the tumor volume was calculated according to the formula [a square of 0.53 × length × width].

Further, in order to study the therapeutic effect of intravenous administration of d12.CALP·ΔRR viral vector against human lung metastatic tumor, 1 × 10⁶ cells of MFH-AI-LM cell line with high metastatic activity to lung isolated from human malignant fibrous histiocytoma MFH-AI cells were injected once into the tail vein of six-week old female athymic nude mice (BALB/c Slc-nu/nu) (Japan SLC), and a lung metastatic tumor model was constructed. Fourteen days after intravenous injection of MFH-AI-LM cells, for histological studies, 100 µl of virus suspension containing 1 × 10⁷ pfu/mouse of d12.CALP·ΔRR viral vector was intravenously injected once by using a 30 gauge needle, and the mice were sacrificed after 13 days. The whole lung metastasized tissues and the brain, liver, kidney, heart, small intestine, uterus and ovary were removed to use as specimens. These specimens were fixed with 2% paraformaldehyde, 0.5% glutaraldehyde, in PBS containing 1 mM MgCl₂ overnight at 4°C. Then, followed by X-Gal staining, the tumors were placed in a substrate solution, containing X-gal (1mg/ml), 5 mM K₃Fe (CN₆) , 5 mM K₄Fe (CN₆) and 1 mM MgCl₂ in PBS for 4 hours at 37° C, and then washed with PBS containing 3% DMSO. The specimens of the whole lung metastasized tissues were fixed in Bouin's solution [15% (v/v) saturated picric acid solution, 1.65% (v/v) formalin, and 1% (v/v) acetic acid/PBS] and embedded in paraffin. Sections of 4 µm thickness were mounted on a poly-L-lysine coated microslide, treated in xylene, and dehydrated through graded concentrations of alcohol solution. Then, hematoxylin-eosin staining was conducted, and the disruption by d12.CALP.ΔRR viral vector in the tumor tissues was observed by using an inverted microscope (Olympus BX-50).

Next, a lung metastatic tumor model was constructed by injecting 1 × 10⁶ or 5 × 10⁵ MFH-AI-LM cells into the tail vein of six-week old female athymic nude mice (BALB/c Slc-nu/nu) (Japan SLC). Seventeen days, 27 days and 34 days after intravenous injection of MFH-AI-LM cells, 50 µl of virus suspension containing 1 × 10⁷ pfu/mouse of d12.CALP.ΔRR viral vector was intravenously injected for three times by using a 30 gauge needle, and the mice were sacrificed after 13 days. The whole lung metastasized tissues were removed, then fixed with 2% paraformaldehyde, 0.5% glutaraldehyde, in PBS containing 1mM MgCl₂ overnight at 4°C. Then, the therapeutic effect by intravenous administration of the d12.CALP.ΔRR viral vector against human lung metastatic tumor was examined.

### A-9 (Statistical analysis)

Statistical differences were determined by using unpaired-Student's t-test. Differences were considered statistically significant with p < 0.05.

### Example B [Results]

### B-1 (Selective replication of a recombinant HSV vector in calponin-positive cells in vitro)

To construct an HSV vector that replicates selectively in calponin-positive cells and proliferating cells, a DNA fragment containing the 4F2 enhancer/-260 calponin promoter/ICP4/IRES-EGFP was inserted into the RR (ICP6) locus (U_{L}36) of the ICP4-deficient HSV mutant d120 (J. Virol. 56, 558-570, 1985) by homologous recombination, and a d12.CALP.ΔRR viral vector was constructed. The d12.CALP.ΔRR viral vector expresses B-galactosidase under the control of an ICP6 promoter, and can express ICP4 protein and EGFP protein under the control of calponin promoter (Figure 1). The calponin-expressing human leiomyosarcoma cell line (SK-LMS-1) and calponin non-expressing human osteosarcoma cell line (OST) were used to evaluate the cell selectivity of the viral replication of d12.CALP.ΔRR viral vector.

Viral titers were assessed by single step growth assays of a multiplicity of infection (MOI) of 0.1 (pfu/cell) (2 × 10⁵ cells/well). The d12.CALP.ΔRR viral vector was replicated in calponin-positive SK-LMS-1 cells but the titers of d12.CALP.ΔRR viral vector decreased in calponin-negative OST cells 72 hours after infection to approximately 1/100000 compared to those of the SK-LMS-1 cells (Figure 2). The rate of proliferation of both cells was at the same level. By conducting immunoblot analysis of the cell extracts 22 hours after the infection, it was found that ICP4 protein was expressed in SK-LMS-1 cells but not in OST cells. This was consistent with the result of viral replication assay. In contrast, d120 viral vector, which is the parental virus of homologous recombination, did not show generation of viral progenies at all in cultures of SK-LMS-1 and OST.

The d12.CALP.ΔRR viral vector was infected to the SK-LMS-1 cells in a 6-well dish, and after 96 hours from the infection, the B-galactosidase expressing cells were stained blue with X-gal agarose overlay, and the expression of EGFP was also examined at the same time with an inverted fluorescence microscope. It was confirmed that B-galactosidase was expressed in the tumor cells that are disrupted and almost abolished, and that EGFP was expressed in the living cells around them (Figure 3). There were a number of observations that both expressions occurred in one cell at the same time.

### B-2 (Sensitivity to ganciclovir, an anti-herpes viral agent, of recombinant HSV-1 vector)

When the d12.CALP.ΔRR viral vector is applied to therapies for human malignant tumors, the most important property is that sensitivity to ganciclovir, an anti-herpes viral agent, is indicated since it has TK genes in an intact state. The d12.CALP.ΔRR viral vector was infected to SK-LMS-1 cells in a 24-well (5 × 10⁴/well) dish, in the presence of ganciclovir of various concentrations (0 to 100 ng/ml), at a multiplicity of infection (MOI) of 0. 01 (pfu/cell). Forty-eight hours after the infection, the cells were stained with X-gal as a substrate, and the number of B-glactosidase-positive plaques per well was counted. Further, the d12.CALP.ΔRR viral vector was infected to Vero E5 cells (2.5 × 10⁵/well) in a 6-well dish in the presence and absence of 1 µg/ml ganciclovir, and 48 hours after the infection, the cells were stained with X-gal as a substrate (Figure 4).

The replication of d12.CALP.ΔRR viral vector was suppressed in the presence of ganciclovir, for SK-LMS-1 cells and Vero E5 cells introduced with ICP4 cDNA. In SK-LMS-1 cells, the replication was completely suppressed in the presence of 40 ng/ml ganciclovir. The d12.CALP.ΔRR viral vector showed sensitivity to ganciclovir, which is equal to replicative HSV-1 mutant hrR3 that is reported to have stronger sensitivity to said drug agent than wild-type virus (Cancer Res. 54, 3963-3966, 2001). This result indicates that the d12.CALP.ΔRR viral vector has a safe measure in which viral infected cells can be eliminated by ganciclovir or acyclovir after therapy.

### B-3 (In vivo treatment and histological analysis)

RT-PCR analysis for total RNA of MFH-AI-LM cell lines was conducted in order to examine whether MFH-AI-LM cell lines express the calponin mRNA, and it was confirmed that MFH-AI-LM cell lines express calponin mRNA (Figure 5a). Further, the MFH-AI-LM cell lines mentioned above were infected with d12.CALP.ΔRR viral vector for 72 hours at a multiplicity of infection (MOI) of 0. 01 (pfu/cell). The replication of the vector was stained with X-gal and evaluated with the plaque formation as an index (Figure 5b). As aresult, it was confirmed that the d12.CALP.ΔRR viral vector was replicated within the MFH-AI-LM cells, and that it shows cytolytic activity against MFH-AI-LM cells. Further, the d12.CALP.ΔRR viral vector was infected to cultured GIST cells and uterine myoma cells for 72 hours, respectively, at a multiplicity of infection (MOI) of 0.01 or 0.1 (pfu/cell). The replication of the vector was stained with X-gal and evaluated with the plaque formation as an index (Figure 6). As a result, it was confirmed that the d12.CALP.ΔRR viral vector is replicated within the cultured GIST cells (Figures 6a, 6b) and uterine myoma cells (Figures 6c, 6d), and from the results of 0.01 MOI (Figures 6a, 6c) and 0.1 MOI (Figures 6b, 6d), it was confirmed that the d12.CALP.ΔRR viral vector shows cytolytic activity in a dose-dependent manner. Particularly, in administration at 0.1 MOI (Figures 6b, 6d), the infection of d12.CALP.ΔRR viral vector to all the tumor cells was observed.

The in vivo anti-tumor effect of the d12.CALP.ΔRR viral vector against subdermally transplanted tumor xenografts that are isolated from MFH-AI cells was examined. The therapeutic effect by one intravenous injection of d12.CALP.ΔRR viral vector against subdermal transplanted tumors of MFH-AI-LM cell lines is expressed as a chronological change (Figure 7). On day 0, the d12.CALP.ΔRR viral vector of 1 × 10⁷ pfu/mouse was infected into the tail vein. The tumor volume (means ± S.E., n = 6) of the group on day 29 after being treated with intravenous injection (d12.CALP. ΔRR viral vector administered) and the non-treated group (PBS administered) were 500 ± 136 mm³ and 183 ± 33 mm³, respectively. The treated group showed significant anti-tumor effect compared to the non-treated group.

The therapeutic effect of d12.CALP.ΔRR viral vector against human lung metastatic tumor by intravenous injection in vivo was examined (Figure 8). The d12.CALP.ΔRR viral vector of 1 × 10⁷ pfu/mouse was injected into the tail vein of a lung metastatic tumor model mouse wherein MFH-AI-LM cells with high metastatic activity to lung isolated from human malignant fibrous histiocytoma MFH-AI cells are used, and metastases tumor in the lung at day 13 (Figures 8a, 8b) and the normal tissues, that is, the brain (Figure 8c), heart (Figure 8d), liver (Figure 8e) excised at the same day were subjected to X-Gal staining. Histological analysis of lung metastatic tumor by hematoxylin-eosin staining (Figures 8f, 8g) was also conducted. By conducting one intravenous administration of d12.CALP.ΔRR viral vector, X-Gal staining which indicates replication of d12.CALP.ΔRR viral vector in the lung metastatic focus and histological tumor necrosis was observed. However, X-Gal staining that indicate the infection and replication of the d12.CALP.ΔRR viral vector in normal tissues such as the brain, heart and liver was not observed.

Subsequently, the therapeutic effect of human lung metastatic tumor wherein the number of MFH-AI-LM cells to be administered are set to 1 × 10⁶ or 5 × 10⁵ , and the d12.CALP.ΔRR viral vector of 1 × 10⁷ pfu/mouse was intravenously injected for a total of three times on day 17, day 27 and day 34 after administration of MFH-AI-LM cells was examined (Figure 9). For all the lung metastatic tumor models constructed by injecting 1 × 10⁶ or 5 × 10⁵ of MFH-AI-LM tumor cells into the tail vein, the lung metastatic tumor-suppressing effect of the groups administered with d12.CALPΔRR vector was apparent. Moreover, the metastasis-suppressing effect of the treated group was also confirmed by the histological analysis by hematoxylin-eosin staining.

### Industrial Applicability

A malignant tumor derived from mesenchymal cells, that is a sarcoma, is resistant to chemotherapy or radiotherapy and continues to relapse even after surgical resection, and by eventually metastasizing to the lung, liver, peritoneum and the like, the prognosis is quite poor. The number of cases in Japan is around 5000 annually, mainly including gastrointestinal stromal tumor (GIST) in the field of digestive surgery, bone or soft tissue sarcoma in the field of orthopedic surgery, and leimyosarcoma in the field of gynecology, malignant mesothelioma in the field of chest/digestive surgery, fibrosarcoma, malignant meningioma, malignant neurinoma in the field of neurosurgery and the like. Although it represents only about 1 to 2% of all cancers, as it generates frequently also to young people, and as there is no effective treatment modality except for some cases that have sensitivity to chemotherapy, the development of a new treatment modality is strongly required socially. For genetic analysis that is associated to the cause and pathology of sarcoma, the mutation of p53 and Rb gene in osteosarcoma and leimyosarcoma, the mutation of KIT gene in GIST, the presence of a fusion gene in Ewing sarcoma and synovial sarcoma and liposarcoma has been reported. However, they have not reached the step of application to therapies. Further, in the animal experiments conducted previously, there have been attempts of direct introduction to sarcoma cells by using various vectors including p53 and cytokine, and herpes simplex virus thyidine kinase (HSV-tk) which is a suicide gene. However, sufficient therapeutical effect has not been obtained.

The gene therapy can increase the cancer cell selectivity at various levels, such as the cell-selective action of genes that are introduced to cancer cells, activity of expression promoters, and infection/introduction of viral vectors, and is focused as a promising therapeutic method also for sarcoma. Indeed, it is reported that osteosarcoma-selective expression of HSV-tk in a non-replicative adenoviral vector by using an osteocalcin promoter can significantly suppress the lung metastatic focus also by intravenous administration (Cancer Gene Ther. 5, 274-280, 1998). However, since osteocalcin is also expressed in normal osteoblasts in the differentiation stage, it is not sufficient to increase the cancer cell-selectivity only by regulating the expression of transgene. In addition, increasing the cell selectivity by promoters of marker genes for differentiation decreases the general purpose of the vector on the other hand. For sarcomas that derive from various tissues and cells and have a limited number of cases, it is not advantageous from the viewpoint of cost-effectiveness of the vector development.

Further, it is impossible to introduce therapeutic genes to all cancer cells with the use of the viral vectors and liposome vectors which are deficient for the replication ability, which have used so far in experimental gene therapies against sarcomas. Therefore, although life extension can be obtained from animal experiments, continuous anti-tumor effect cannot be expected. Moreover, if the introduction efficiency of genes to cancer cells is low, the more the number of viral vectors will be needed, and the risk of induction of excessive immunoreaction and allergic reaction will increase.

For treatment of intractable sarcomas, it has been believed that a completely novel approach that is different from that of the conventional methods is needed, however, no clues for such approach was obtained. The examples of the present description can respond to such requirements, and the present description can provide a cell-specific expression/replication vector that does not act to normal cells, which replicates in particular cells such as malignant tumor cells and the like, not limited to sarcoma, and expresses specifically therapeutic genes while disrupting tumor cells. By using said cell-specific expression/replication vector provided with a safety measure that can stop the replication of virus with a drug agent after the completion of the therapy, a gene therapy using a cell-specific expression/replication vector for human can be possible for the first time in the world.

Calponin gene is expressed mainly in smooth muscle cells in adult body, and particularly, since the proliferation of vascular smooth muscle cells is the cause of proliferating vascular lesion such as tumor neoangiogenesis and blood vessel constriction after stent placement and diabetic retinopathy, therapies with these diseases becomes possible by selectively disrupting the proliferating smooth muscle cells with the use of the smooth muscle cell-specific expression/replication vector having a calponin promoter provided from the present description. Among these, the therapeutic method which selectively disrupts the tumor vascular smooth muscles (included for example only), which becomes possible for the first time by the present description, has a possibility to present an innovative effect as a therapeutic method for cancer that is effective to all solid cancers. Further, it can effectively act as a therapeutic agent against proliferating glomerulonephritis which is caused by the proliferation of mesangial cells that express calponin, or fibrosis such as pulmonary fibrosis and hepatic fibrosis which is caused by the proliferation of myofibroblast that express calponin.

## Claims

1. A herpes simplex virus (HSV) vector which induces viral gene expression and viral replication specifically in a cell expressing calponin and proliferating, which is not replicated in adult normal cells, and which is capable of suppressing its replication at a desired timing by using the thymidine kinase gene,
wherein the vector comprises a DNA fragment comprising:
(i) a promoter region of the human calponin gene comprising the nucleotide sequence shown by SEQ ID No: 3;
(ii) the ICP4 gene encoding a transcription factor essential for initiation of a herpes viral replication which is integrated downstream of the promoter region of the human calponin gene;
(iii) the EGFP (enhanced green fluorescent protein) gene linked to the downstream of the ICP4 gene via an internal ribosomal entry site; and
(iv) the LacZ gene which is integrated upstream of said promoter region of the human calponin gene
and wherein this DNA fragment is inserted by recombination into the ribonucleotide reductase gene locus of a HSV vector that comprises an endogenous thymidine kinase gene and lacks function of the endogenous ICP4 gene,
and wherein the expression of both the LacZ gene and the EGFP gene integrated in the vector are used as markers to identify the recombinant HSV vector.

2. The HSV vector according to claim 1, wherein an enhancer is integrated upstream of the promoter region of the human calponin gene.

3. The HSV vector according to claim 2, wherein the enhancer is a 4F2 enhancer.

4. A therapeutic drug comprising the HSV vector according to any one of claims 1 to 3.

5. A method for producing an HSV vector according to any one of claims 1 to 4 which vector induces viral gene expression and viral replication specifically in a cell expressing calponin and proliferating, which is not replicated in adult normal cells, and which is capable of suppressing its replication at a desired timing by using the thymidine kinase gene, the method comprising the steps of:
(a) preparing a DNA fragment comprising,
(i) a promoter region of the human calponin gene comprising the nucleotide sequence shown by SEQ ID No. 3,
(ii) the ICP4 gene encoding a transcription factor essential for initiation of a herpes viral replication which is integrated downstream of the promoter region of the human calponin gene,
(iii) the EGFP gene linked to the downstream of the ICP4 gene via an internal ribosomal entry site, and
(iv) the LacZ gene integrated upstream the promoter region of the human calponin gene;
(b) preparing recombinants by cotransfection with the HSV vector that comprises an endogenous thymidine kinase gene and lacks function of the endogenous ICP4 gene together with the DNA fragment into a cell in which a promoter region of the human calponin gene that comprises the nucleotide sequence shown by SEQ ID No: 3 can be activated or a cell which expresses the human calponin gene; and
(c) in order to screen a recombinant HSV vector wherein the DNA fragment is inserted by a homologous recombination into the ribonucleotide reductase gene locus of the HSV vector that comprises an endogenous thymidine kinase gene and lacks function of the endogenous ICP4 gene from among the recombinants, selecting a single clone of the HSV vector from the recombinants by limiting dilution without using agarose overlay assay using the expressions of both the LacZ gene and the EGFP gene as markers.

## Patentansprüche

1. Herpes simplex-Virus (HSV)-Vektor, der spezifisch eine virale Genexpression und virale Replikation in einer Calponin exprimierenden und proliferierenden Zelle induziert, der nicht in normalen Zellen eines Erwachsenen repliziert wird und der in der Lage ist, unter Verwendung des Thymidinkinase-Gens seine Replikation zu einem gewünschten Zeitpunkt zu unterdrücken,
wobei der Vektor ein DNA-Fragment umfasst, weiches folgendes umfasst:
(i) eine Promotorregion des humanen Calponin-Gens, die die in SEQ ID No. 3 gezeigte Nukleotidsequenz umfasst,
(ii) das ICP4-Gen, das einen für die Auslösung einer Herpesvirus-Replikation notwendigen Transkriptionsfaktor codiert, das stromabwärts der Promotorregion des humanen Calponin-Gens eingefügt ist,
(iii) das EGFP (enhanced green fluorescent protein - verstärkt grün fluoreszierendes Protein)-Gen, das über eine interne ribosomale Eintrittsstelle mit dem stromabwärts gelegenen Teil des ICP4-Gens verknüpft ist, und
(iv) das LacZ-Gen, das stromaufwärts der Promotorregion des humanen Calponin-Gens eingebaut ist,
und wobei dieses DNA-Fragment durch Rekombination in den Locus des Ribonucleotidreductase-Gens eines HSV-Vektors eingebaut ist, der ein endogenes Thymidinkinase-Gen umfasst und dem die Funktion des endogenen ICP4-Gens fehlt,
und wobei die Expression von sowohl dem LacZ-Gen als auch dem EGFP-Gen, die in den Vektor eingebracht sind, als Marker zur Identifizierung des rekombinanten HSV-Vektors verwendet werden.

2. HSV-Vektor gemäß Anspruch 1, wobei ein Enhancer (Verstärker) stromaufwärts der Promotorregion des humanen Calponin-Gens eingebaut ist.

3. HSV-Vektor gemäß Anspruch 2, wobei der Enhancer ein 4F2-Enhancer ist.

4. Therapeutischer Wirkstoff, der den HSV-Vektor gemäß einem der Ansprüche 1 bis 3 umfasst.

5. Verfahren zur Herstellung eines HSV-Vektors gemäß einem der Ansprüche 1 bis 4, der spezifisch in einer Calponin exprimierenden und proliferierenden Zelle virale Genexpression und virale Replikation induziert, der in normalen Zellen eines Erwachsenen nicht repliziert wird und der in der Lage ist, unter Verwendung des Thymidinkinase-Gens seine Replikation zu einem gewünschten Zeitpunkt zu unterdrücken, wobei das Verfahren die folgenden Stufen umfasst:
(a) Herstellen eines DNA-Fragments, das folgendes umfasst,
(i) eine Promotorregion des humanen Calponin-Gens, die die in SEQ ID No. 3 gezeigte Nukleotidsequenz umfasst,
(ii) das ICP4-Gen, das einen für die Auslösung einer Herpesvirus-Replikation notwendigen Transkriptionsfaktor codiert, das stromabwärts der Promotorregion des humanen Calponin-Gens eingefügt ist,
(iii) das EGFP-Gen, das über eine interne ribosomale Eintrittsstelle mit dem stromabwärts gelegenen Teil des ICP4-Gens verknüpft ist, und
(iv) das LacZ-Gen, das stromaufwärts der Promotorregion des humanen Calponin-Gens eingebaut ist,
(b) Herstellen von Rekombinanten, indem der HSV-Vektor, der ein endogenes Thymdidinkinase-Gen aufweist und dem die Funktion des endogenen ICP4-Gens fehlen, zusammen mit dem DNA-Fragment in eine Zelle co-transfiziert werden, in der eine Promotorregion des humanen Calponin-Gens, das die in SEQ ID No. 3 gezeigte Nukleotidsequenz umfasst, aktiviert werden kann, oder in eine Zelle, die humanes Calponin-Gen exprimiert, und
(c) Auswählen eines einzelnen Klons des HSV-Vektors aus den Rekombinanten durch Limiting Dilution (Grenzverdünnung) ohne Verwendung eines Agarose-Overlay-Tests, unter Verwendung der Expression von sowohl dem LacZ-Gen als auch dem EGFP-Gen als Marker, um einen rekombinanten HSV-Vektor aus den Rekombinanten zu überprüften, bei dem das DNA-Fragment durch homologe Rekombination in den Locus des Ribonudeotidreductase-Gens des HSV-Vektors eingefügt ist, der ein endogenes Thymidinkinase-Gen umfasst und dem die Funktion des endogenen ICP4-Gens fehlt.

## Revendications

1. Vecteur du virus herpès simplex (HSV) qui induit une expression génique virale et une réplication virale spécifiquement dans une cellule exprimant la calponine et proliférant, qui n'est pas répliqué dans des cellules adultes normales, et qui est capable d'arrêter sa réplication à un moment souhaité en utilisant le gène de la thymidine kinase,
où le vecteur comprend un fragment d'ADN comprenant
(i) une région promoteur du gène de la calponine humaine comprenant la séquence nucléotidique représentée par SEQ ID NO : 3 ;
(ii) le gène ICP4 codant pour un facteur de transcription essentiel à l'initiation d'une réplication de l'herpès virus qui est intégré en aval de la région promoteur du gène de la calponine humaine ;
(iii) le gène EGFP (protéine fluorescente verte amplifiée) lié en aval du gène ICP4 par l'intermédiaire d'un site interne d'entrée des ribosomes ; et
(iv) le gène LacZ qui est intégré en amont de ladite région promoteur du gène de la calponine humaine
et où ce fragment d'ADN est inséré par recombinaison dans le locus d'un gène de ribonucléotide réductase d'un vecteur HSV qui comprend un gène de thymidine kinase endogène et qui manque de la fonction du gène ICP4 endogène,
et où les expressions à la fois du gène LacZ et du gène EGFP intégrés dans le vecteur sont utilisées en tant que marqueurs pour identifier le vecteur HSV recombinant.

2. Vecteur HSV selon la revendication 1, dans lequel un amplificateur est intégré en amont de la région promoteur du gène de la calponine humaine.

3. Vecteur HSV selon la revendication 2, dans lequel l'amplificateur est un amplificateur 4F2.

4. Médicament thérapeutique comprenant le vecteur HSV selon l'une quelconque des revendications 1 à 3.

5. Procédé de production d'un vecteur HSV selon l'une quelconque des revendications 1 à 4, lequel vecteur induit une expression génique virale et une réplication virale spécifiquement dans une cellule exprimant la calponine et proliférant, qui n'est pas répliqué dans des cellules adultes normales, et qui est capable d'arrêter sa réplication à un moment souhaité en utilisant le gène de la thymidine kinase, le procédé comprenant les étapes consistant à :
(a) préparer un fragment d'ADN comprenant,
(i) une région promoteur du gène de la calponine humaine comprenant la séquence nucléotidique représentée par SEQ ID NO : 3,
(ii) le gène ICP4 codant pour un facteur de transcription essentiel à l'initiation d'une réplication de l'herpès virus qui est intégré en aval de la région promoteur du gène de la calponine humaine,
(iii) le gène EGFP lié en aval du gène ICP4 par l'intermédiaire d'un site interne d'entrée des ribosomes, et
(iv) le gène LacZ intégré en amont de ladite région promoteur du gène de la calponine humaine ;
(b) préparer des recombinants par cotransfection avec le vecteur HSV qui comprend un gène de thymidine kinase endogène et qui manque de la fonction du gène ICP4 endogène conjointement avec le fragment d'ADN dans une cellule dans laquelle une région promoteur du gène de la calponine humaine qui comprend la séquence nocléotidique représentée par SEQ ID NO : 3 peut être activée ou une cellule qui exprime le gène de la calponine humaine ; et
(c) afin de cribler un vecteur HSV recombinant dans lequel le fragment d'ADN est inséré par une recombinaison homologue dans le locus d'un gène de ribonucléotide réductase du vecteur HSV qui comprend un gène de thymidine kinase endogène et qui manque de la fonction du gène ICP4 endogène, parmi les recombinants, en sélectionnant un clone unique du vecteur HSV parmi les recombinants par dilution limitante sans utiliser de test sur couche d'agarose en utilisant les expressions à la fois du gène LacZ et du gène EGFP en tant que marqueur.
